(19) Europäisches Patentamt
European Patent Office
Office européen des brevets

(11) **EP 2 568 980 B1**

(12) # EUROPEAN PATENT SPECIFICATION


(45) Date of publication and mention of the grant of the patent: **11.11.2015 Bulletin 2015/46**

(21) Application number: **11731566.3**

(22) Date of filing: **12.05.2011**

(51) Int Cl.:
***A61K 31/365*** *(2006.01)* ***A61K 31/425*** *(2006.01)*
***A61K 31/7048*** *(2006.01)* ***A61K 9/00*** *(2006.01)*
***A61P 33/00*** *(2006.01)* ***A61P 33/10*** *(2006.01)*
***A61K 45/06*** *(2006.01)* ***A61K 47/18*** *(2006.01)*
***A61K 47/26*** *(2006.01)* ***A61K 47/44*** *(2006.01)*

(86) International application number: **PCT/US2011/036321**

(87) International publication number: **WO 2011/143479 (17.11.2011 Gazette 2011/46)**


(54) **INJECTABLE PARASITICIDAL FORMULATIONS OF LEVAMISOLE AND MACROCYCLIC LACTONES**

INJIZIERBARE PARASITIZIDE FORMULIERUNGEN AUS LEVAMISOL UND MAKROCYCLISCHEN LACTONEN

FORMULATIONS PARASITICIDES INJECTABLES DE LÉVAMISOLE ET DE LACTONES MACROCYCLIQUES


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2010 US 333882 P**

(43) Date of publication of application: **20.03.2013 Bulletin 2013/12**



(73) Proprietor: **Merial, Inc.**
**Duluth, GA 30096 (US)**

(72) Inventors:
- **HOLMES, Robert**
  **Biirkenhead, Auckland (NZ)**
- **RAZZAK, Majid**
  **Glenfield, Auckland (NZ)**
- **JOHNSON, Alan**
  **Papatoetoe (NZ)**
- **GOSWAMI, Jitendra**
  **Cumbria Downs, Howick (NZ)**
- **AWASTHI, Atul**
  **Manukau City (NZ)**

(74) Representative: **D Young & Co LLP**
**120 Holborn**
**London EC1N 2DY (GB)**



(56) References cited:
**WO-A1-00/74489** **WO-A1-2004/009080**
**WO-A1-2004/069242** **WO-A2-2007/068073**
**US-A- 4 389 397** **US-B1- 6 174 540**

- **SARI PEYAMI ET AL: "HPLC assay of levamisole and abamectin in sheep plasma for application to pharmacokinetic studies", JOURNAL OF LIQUID CHROMATOGRAPHY & RELATED TECHNOLOGIES, vol. 29, no. 15, 2006, pages 2277-2290, XP009151151, ISSN: 1082-6076**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### INCORPORATION BY REFERENCE

This application claims priority to U.S. provisional patent application No. 61/333,882, filed May 12, 2010. All documents cited or referenced in the applicant cited documents, and all documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention.

### FIELD OF THE INVENTION

The present invention is directed to new injectable parasiticidal formulations comprising levamisole and macrocyclic lactones. The present invention also provides methods for eradicating, controlling, and preventing parasite infestation and/or infection in birds, fish and mammals. The formulations of the invention may be administered to animals, particularly mammals, fish and birds, to prevent or treat parasitic infestation and/or infection.

### BACKGROUND OF THE INVENTION

Animals, such as mammals, fish and birds are often susceptible to parasite infestation and/or infection. These parasites may be ectoparasites, such as insects and acarine species, and endoparasites such as filariae and other worms. Endoparasites pose a significant problem to those who raise production animals such as bovines, ovines, and porcines. Thus, there is an ongoing need to develop active pesticidal and parasiticidal compounds to protect animals against attack or infestation/infection by pests.

Many endoparasiticides or anthelmintics exist in the art for treating internal parasites and other animal pests. These endoparasiticides vary in cost as well as their effectiveness to a particular parasite. However, the results of treatment with these pesticides are not always satisfactory because of continued development of resistance by the parasite to the available therapeutic agents. Thus, there is a need in the art for more effective parasiticidal agents for treatment and protection of animals, e.g. mammals, fish and birds from infestation by animal parasites.

The avermectin and milbemycin series of compounds are potent anthelmintic and antiparasitic agents against a wide range of internal and external parasites. The compounds which belong to this series are either natural products or are semi-synthetic derivatives thereof. The structure of these two series of compounds are closely related and they both share a complex 16-membered macrocyclic lactone ring; however, the milbemycins do not contain the disaccharide substituent in the 13-position of the lactone ring. The natural product avermectins are disclosed in U.S. Patent No. 4,310,519 to Albers-Schonberg, et al., and the 22, 23-dihydro avermectin compounds are disclosed in Chabala, et al., U.S. Patent No. 4,199,569. For a general discussion of avermectins, which include a discussion of their uses in humans and animals, see "Ivermectin and Abamectin," W. C. Campbell, ed., Springer-Verlag, N.Y. (1989). Naturally occurring milbemycins are described in Aoki et al., U.S. Patent No. 3,950,360 as well as in the various references cited in "The Merck Index" 12th ed., S. Budavari, Ed., Merck & Co., Inc. Whitehouse Station, N.J. (1996). Semi-synthetic derivatives of these classes of compounds are well known in the art and are described, for example, in U.S. Patent No. 5,077,308, U.S. Patent No. 4,859,657, U.S. Patent No. 4,963,582, U.S. Patent No. 4,855,317, U.S. Patent No. 4,871,719, U.S. Patent No. 4,874,749, U.S. Patent No. 4,427,663, U.S. Patent No. 4,310,519, U.S. Patent No. 4,199,569, U.S. Patent No. 5,055,596, U.S. Patent No. 4,973,711, U.S. Patent No. 4,978,677, and U.S. Patent No. 4,920,148, all incorporated herein by reference in their entirety. A particularly useful macrocyclic lactone is eprinomectin, which is described in US6,174,540, US6,733,767, US5,602,107 to Merck (all incorporated by reference). This compound provides for a very short milk withdrawal period due to it tendency to partition away from the milk of a lactating mammal.

To overcome the problem of resistance, various combinations of anthelmintics have been extensively researched. Unfortunately, combinations of macrocyclic lactone anthelmintics with anthelmintics of other families have proven difficult to formulate at high concentration and with an acceptable viscosity for injection. For example, Closamectin (manufactured by Norbrook) contains ivermectin at 0.5% and closantel at 12.5% and is so viscous that the product is supplied in a special pack that has a warming device. The warming device is activated 15 minutes before administration and the pack requires periodic shaking throughout this time to ensure temperature reduces the viscosity sufficiently for acceptable administration.

Another example, which has been the subject of considerable research, is combining a macrocyclic lactone (ML) with levamisole. The difficulties of combining these two actives lies in the stability of each being incompatible in the environment in which the other is stable. Levamisole is stable in acid conditions usually a pH of about 4 or below, while macrocyclic lactones are unstable in conditions where the pH is acidic. Both levamisole and macrocyclic lactones are well known in the art. In particular, levamisole has been used as an anthelmintic to treat nematodes and respiratory worm infestations in both humans and animals. Levamisole may be combined with other actives, although often with significant difficulty, as is described in various patents and applications. For example, US 2009/0075918 A1 to Neto et al. appears to contemplate adding various organic salts of levamisole to macrocyclic lactones, but provides no working examples. Other applications/documents that mention levamisole in combination with macrocyclic lactones include WO 00/74489, Sari et al., Journal of liquid chromatography and related technologies. Vol. 29, no. 15, 2006, pages 2277-2290, WO 2007/068073; WO 2004/069242; WO 00/061068, which describes non-aqueous pour-on formulations that further contain triclabendazole, and WO 04/009080, which describes stable pyrrolidone-based formulations of avermectins or milbemycins combined with levamisole. Even more recently, WO2010021555 (to Intervet) has described oral drenches containing levamisole and a macrocyclic lactone, where the macrocyclic lactone is apparently stabilized via the inclusion of a protective agent, namely hydroxypropyl starch phosphate. Since this type of "protective agent" also serves as a thickener and emulsion stabilizer, this particular approach to stabilizing the macrocyclic lactone component may be ill-suited to developing injectable formulations. Hydroxypropyl starch phosphate (HP starch) is typically used for creams & lotions, foundations, mascara, color cosmetics, liquid make-up, liquid talc, conditioners, shampoos, sunscreen products, and antiperspirants. Finally, AU200310102 (to Nufarm and Novartis) describes formulations having levamisole, avermectins, and benzimidazole, said formulations appearing to make use of a biphasic solvent system, in an attempt to overcome the problematic instability of formulations which comprise levamisole and macrocyclic lactones. Several patents describe long-acting injectable combination formulations comprising macrocyclic lactones and other actives, for example US patent numbers US 6,174,540 and US 6,733,767 (to Merck), which use castor oil and liquid polymers, respectively, to achieve the sustained release of the macrocyclic lactones and the other actives. US 4,381,397 describes injectable formulations of ivermectin. After reviewing the current literature, applicants are unaware of any previously described, effective combinations of levamisole and eprinomectin, injectable or otherwise.

It is expressly noted that citation or identification of any document in this application is not an admission that such document is available as prior art to the present invention. Any foregoing applications, and all documents cited therein or during their prosecution ("application cited documents") and all documents cited or referenced in the application cited documents, and all documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention.

## SUMMARY OF THE INVENTION

The invention provides novel parasiticidal formulations comprising levamisole and macrocyclic lactones, particularly avermectins such as ivermectin and eprinomectin, and milbemycins, such as moxidectin, milbemectin, and milbemycin oxime. The formulations are storage-stable and provided in a single solvent system that is miscible in water. Therefore, the formulations according to the present invention may be classified as micellar solutions as opposed to emulsions or biphasic emulsions. The invention also provides methods for the treatment and prevention of parasitic infestation and/or infection of animals.

The inventive formulations comprising the levamisole and macrocyclic lactones are highly effective for the treatment or prophylaxis of parasites in or on mammals, fish and birds, and in particular, cats, dogs, horses, chickens, pigs, sheep and cattle with the aim of ridding these hosts of all the parasites commonly encountered by mammals, fish and birds. The invention also provides for effective and long-lasting defense against endoparasites, such as helminths, nematodes, filariae, hookworms, whipworms and roundworms of the digestive tract of animals and humans.

Accordingly, in a first aspect the present invention provides a pharmaceutically or veterinarily acceptable formulation adapted to be injected into an animal, which formulation comprises;

a. an effective amount of a levamisole;
b. an effective amount of at least one macrocyclic lactone;
c. a pharmaceutically acceptable system of solvents and surfactants wherein the system provides for acceptable storage stability for both the levamisole and the macrocyclic lactone and wherein the system provides for a viscosity which is suitable for injection;

wherein the system comprises at least one· surfactant, wherein the surfactant is CREMAPHOR EL,
and a water miscible organic solvent which is dimethylacetamide (DMA).

In a second aspect, the present invention provides a pharmaceutically or veterinarily acceptable formulation adapted to be injected into an animal, which formulation comprises; ·

a. an effective amount of a levamisole;

b. an effective amount of at least one macrocyclic lactone;

c. a pharmaceutically acceptable system of solvents and surfactants wherein the system provides for acceptable storage stability for both the levamisole and the macrocyclic lactone and wherein the system provides for a viscosity which is suitable for injection;

wherein the system comprises at least one surfactant, wherein the surfactant is CREMAPHOR EL, and a water miscible organic solvent which is dimethylacetamide (DMA), wherein the macrocyclic lactone is eprinomectin and its concentration is about 0.5% to about 0.9% (w/v); the levamisole is levamisole phosphate and its concentration is about 18% to about 25% (w/v); the surfactant is POLYSORBATE 80, CREMAPHOR EL, or combinations thereof, and its concentration is about 3% to about 7% (w/v); the antioxidant is BHT, the solvent is DMA and its concentration is about 1% to about 10% (w/v); and the preservative is methylparaben.

In a third aspect, the present invention provides a formulation according to the first and second aspects or use in preventing or treating internal parasite infestations in animals.

In a fourth aspect, the present invention provides a method for producing the formulation according to claim 1 or 2 comprising the steps of:

a. adding water for injection to a vessel,

b. dissolving the Levamisole in the water,

c. in a separate vessel dissolving Eprinomectin and BHT in DMA,

d. adding the surfactant to the DMA/Eprinomectin blend,

e. transferring the EprinomectinfDMA·blend to the bulk aqueous phase with mixing, and

f. optionally, bringing the batch to volume with added WFI and mix.

It is an object of the invention to not encompass within the invention any previously known product, process of making the product, or method of using the product such that the Applicants reserve the right to this invention and hereby disclose a disclaimer of any previously known product, process, or method.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law; e.g., they allow for elements not explicitly recited, but exclude elements that are found in the prior art or that affect a basic or novel characteristic of the invention.

These and other embodiments are disclosed or are obvious from, and encompassed by, the following Detailed Description.

## BRIEF DESCRIPTION OF DRAWINGS

The following Detailed Description, given by way of example, and not intended to limit the invention to specific embodiments described, may be understood in conjunction with the accompanying Figures, incorporated herein by reference, in which:

FIG. 1 is a flow diagram outlining the preparation of formulations according to the instant invention

FIG. 2 depicts levamisole plasma concentration at time points after administration of formulations according to the instant invention

FIG. 3 depicts eprinomectin plasma concentration at time points after administration of formulations according to the instant invention

## DETAILED DESCRIPTION

The present invention provides novel injectable levamisole / macrocyclic lactone formulations with parasiticidal activity, for the treatment or prevention of parasitic infestations and/or infection in an animal.

An important aspect of formulations containing eprinomectin as the macrocyclic lactone component is that said formulations may have shortened withdrawal times before it is appropriate to obtain milk from lactating mammals. This is because of the macrocyclic lactones, eprinomectin has relatively the lowest milk to plasma partition ratio (-0.2). For a point of reference and definition, a milk to plasma partition ratio of 1 would indicate the active tends to distribute evenly between milk and plasma.

Formulations described herein are particularly effective for controlling endoparasites. Endoparasites include, but are not limited to, nematodes (such as roundworms, hookworms, whipworms and heartworms) and cestodes (tapeworms) and trematodes (flukes). Therefore, the inventive formulations have substantial utility in preventing damage to crops, plants, plant propagation material and wood-containing property, and in controlling and preventing the infestation and/or infection of animals by parasites.

Also contemplated within the scope of the invention are acid or base salts of the compounds in the compositions of the invention, where applicable.

The term "acid" contemplates all pharmaceutically acceptable inorganic or organic acids. Inorganic acids include mineral acids such as hydrohalic acids such as hydrobromic acid and hydrochloric acid, sulfuric acid, phosphoric acids and nitric acid. Organic acids include all pharmaceutically acceptable aliphatic, alicyclic and aromatic carboxylic acids, dicarboxylic acids, tricarboxylic acids and fatty acids. In one embodiment of the acids, the acids are straight chain or branched, saturated or unsaturated $C_1$-$C_{20}$ aliphatic carboxylic acids, which are optionally substituted by halogen or by hydroxyl groups, or $C_6$-$C_{12}$ aromatic carboxylic acids. Examples of such acids are carbonic acid, formic acid, acetic acid, propionic acid, isopropionic acid, valeric acid, $\alpha$-hydroxy acids such as glycolic acid and lactic acid, chloroacetic acid, benzoic acid, methane sulfonic acid, and salicylic acid. Examples of dicarboxylic acids include oxalic acid, malic acid, succinic acid, tartaric acid, fumaric acid, and maleic acid. An example of a tricarboxylic acid is citric acid. Fatty acids include all pharmaceutically acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic acid, linolenic acid, and phenylsteric acid. Other acids include gluconic acid, glycoheptonic acid and lactobionic acid.

The term "base" contemplates all pharmaceutically acceptable inorganic or organic bases, including hydroxides, carbonates or bicarbonates of alkali metal or alkaline earth metals. Salts formed with such bases include, for example, the alkali metal and alkaline earth metal salts, including, but not limited to, as the lithium, sodium, potassium, magnesium or calcium salts. Salts formed with organic bases include the common hydrocarbon and heterocyclic amine salts, which include, for example, ammonium salts ($NH4^+$), alkyl- and dialkylammonium salts, and salts of cyclic amines such as the morpholine and piperidine salts.

In addition, the compounds within the compositions of the invention may exist as hydrates or solvates, in which a certain stoichiometric amount of water or a solvent is associated with the molecule in the crystalline form. The compositions of the invention may include hydrates and solvates of the active agents.

Definitions:

Terms used herein will have their customary meanings in the art unless specified. As used herein the term "anthelmintic" and variations thereof encompasses one or more nematocidal, trematocidal and cestocidal active compounds. The term "pesticidal" and variations thereof includes any said anthelmintic, any endectoparasiticidal, and/or any ectoparasiticidal compound. Where the context allows the term "ectoparasiticidal" includes compounds effective against any one or more ectoparasites including ticks, lice, fleas, mites and the like. Further, as used herein the "endectoparasiticidal" and variants thereof includes compounds and/or formulations that are active against internal (endo) and external (ecto) parasites.

As used herein the term "solubility" refers to the ability of a compound to be dissolved in a specific phase; and "lipophilic" means a greater tendency to an organic, oil or the like phase as opposed to another phase (for example, the aqueous phase).

As referred to herein the term "injectable" in the context of fluids or liquids covers viscosities ranging from a free flowing liquid to a thin gel consistency that is capable of being expelled by syringe and suitable for being administered to an animal via injection. As used herein, a suitable or appropriate viscosity for injection may be between 1-50 cp or from 1-10 cp. Routes of injection may be parenteral, for example intramuscular (IM), intraperitoneal (IP), or subcutaneous (SQ).

As referred to herein the term "acceptable storage stability" means stable for greater than 3 months at room temperature.

Formulations of the Invention:

The formulations of the invention comprise at least levamisole and at least one macrocyclic lactone active compound, and are provided as micellar solutions of actives, which are miscible in water for injection, and are particularly effective at controlling / combating parasites, particularly endoparasites. In certain embodiments, the formulations of the invention are useful in veterinary applications, including for controlling parasites in or on an animal.

In one embodiment, the at least one macrocyclic lactone active agent in the formulations of the invention may be at least one avermectin or milbemycin compound. In various embodiments, the at least one macrocyclic lactone compound may be abamectin, avermectin, dimadectin, doramectin, emamectin, eprinomectin, ivermectin, latidectin, lepimectin, selamectin, milbemectin, milbemycin D, milbemycin oxime, moxidectin or nemadectin.

In another embodiment, the at least one macrocyclic lactone active agent may be any one of the avermectins or avermectin monosaccharides modified in the 4' or 4" position described in US 7,704,961, US 7,521,429, US 2006-0105970 A1, US 7,678,740, US 7,632,820, US 7,678,773, US 7,605,134, and US 6,933,260 (each to Merial). It will be appreciated by a skilled person that any of the above-recited avermectins exhibiting a relatively low milk to plasma partition ratio (for example less than about 1, less than about 0.5, and even more favorably, less than about 0.2) will be a particularly desirable component of formulations according to the instant invention.

In an embodiment, the levamisole is levamisole phosphate and is present in an amount sufficient to deliver a dose of at least 4 mg/kg animal bodyweight. In another embodiment, the macrocyclic lactone is either ivermectin or eprinomectin and is present in an amount sufficient to deliver a dose of at least 150 μg/kg animal bodyweight.

In a particular embodiment, the present invention is a stable injectable formulation comprising eprinomectin and levamisole and is suitable for use in cattle. In an embodiment, the formulation is an injectable combination anthelmintic containing about 7 g/L eprinomectin and about 223 g/L levamisole phosphate.

In an emodiment, the formulation requires not more than a 35 day withholding period for either milk or meat.

In an embodiment, the formulation is administered at a dose rate of 1 mL per 35 kg (200μg eprinomectin and 6.4 mg levamisole phosphate per kg, equivalent to 5 mg levamisole HCl per kg). In another embodiment, the formulation is effective against a broad range of internal parasites including *Ostertagla spp*., *Trichostrongylus axel, Cooperia spp*., suckling lice, and certain strains resistant to certain macrocyclic lactones and/or benzamidazoles.

In an embodiment, the formulations have a shelf life and storage condition of at least 2 years at 2-6 °C. In another embodiment, the formulations are prepared as pack sizes of 100 to 1000 mL. In a particular embodiment, the pack sizes are 500 mL flexible packages, or flexipacks.

In another preferred embodiment, the formulations of the invention will comprise a pharmaceutically acceptable amide including, but not limited to, dimethylformamide, dimethylacetamide, 2-pyrrolidone, N-methylpyrrolidone and the like.

Surfactants are well known in the art and may include non-ionic surfactants, cationic surfactants and anionic surfactants. Anionic surfactants include, but are not limited to, alkaline stearates (e.g. sodium, potassium or ammonium stearate); calcium stearate or triethanolamine stearate; sodium abietate; alkyl sulfates, which include but are not limited to sodium lauryl sulfate and sodium cetyl sulfate; sodium dodecylbenzenesulphonate or sodium dioctyl sulphosuccinate; or fatty acids (e.g. coconut oil);

Cationic surfactants include, but are not limited to, any known water-soluble quaternary ammonium salts such as cetyltrimethylammonium bromide and the like; and known amine salts such as octadecylamine hydrochloride and the like.

Non-ionic surfactants include, but are not limited to, optionally polyoxyethylenated esters of sorbitan, e.g. Polysorbate 80, or polyoxyethylenated alkyl ethers; polyethylene glycol stearate, polyoxyethylenated derivatives of castor oil, polyglycerol esters, polyoxyethylenated fatty alcohols, polyoxyethylenated fatty acids or copolymers of ethylene oxide and of propylene oxide. Other surfactants that are suitable for the formulations of the present invention include amphoteric surfactants, such as substituted lauryl compounds of betaine. The surfactant of the inventive formulations may also be a mixture of at least two different surfactants.

In another embodiment, the levamisole is levamisole phosphate and the macrocyclic lactone is either eprinomectin or ivermectin. Thus in an embodiment, the formulation of the present invention may comprise:

(a) Levamisole phosphate;
(b) Eprinomectin, ivermectin, abamectin, doramectin or moxidectin;
(c) DMA
(d) CREMOPHOR EL; and
(e) WFI

The inventive formulations may contain other inert ingredients such as antioxidants, preservatives, or pH stabilizers. These compounds are well known in the formulation art. Antioxidant such as an alpha tocopherol, ascorbic acid, ascrobyl palmitate, fumaric acid, malic acid, sodium ascorbate, sodium metabisulfate, n-propyl gallate, BHA (butylated hydroxy anisole), BHT (butylated hydroxy toluene) monothioglycerol and the like, may be added to the present formulation. In certain embodiments, the antioxidants are generally added to the formulation in amounts of from about 0.01 to about 2.0%, based upon total weight of the formulation, with about 0.05 to about 1.0% being especially preferred.

In some embodiments, preservatives, such as the parabens (methylparaben and/or propylparaben), are suitably used in the formulation in amounts ranging from about 0.01 to about 2.0%, with about 0.05 to about 1.0% being especially preferred. Other preservatives include benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, and the like. Preferred ranges for these compounds include from about 0.01 to about 5%.

Compounds which stabilize the pH of the formulation are also contemplated and may be used in certain embodiments of the inventive formulations. Again, such compounds are well known to a practitioner in the art as well as how to use these compounds. Buffering systems include, for example, systems selected from the group consisting of acetic acid/acetate, malic acid/malate, citric acid/citrate, tataric acid/tartrate, lactic acid/lactate, phosphoric acid/phosphate, glycine/glycimate, tris, glutamic acid/glutamates and sodium carbonate.

Parasites Controlled:

The injectable levamisole / macrocyclic lactone formulations are particularly effective for efficiently controlling endoparasites, such as flukes, hookworms, and helminths such as cestodes, nematodes, and trematodes. Endoparasites further include helminths such as *Anaplocephala,*

*Ancylostoma, Anecator, Ascaris, Capillaria, Cooperia, Dipylidium, Dirofilaria, Echinococcus, Enterobius, Fasciola, Haemonchus*, *Oesophagostumum*, *Ostertagia*, *Toxocara, Strongyloides, Toxascaris, Trichinella*, *Trichuris*, and *Trichostrongylus*. Or others from the class of helminths, such as from the class of helminths, for example, *Arrcylostoma duodenale*, *Ancylostoma ceylanicum, Acylostoma braziliensis, Ancylostoma* spp., *Ascaris lubricoides, Ascaris* spp., *Brugia malayi, Brugia timori, Bunostomum* spp., *Chabertia* spp., *Clonorchis* spp., *Cooperia* spp., *Dicrocoelium* spp, *Dictyocaulus filaria, Diphyllobothrium latum, Dracunculus medinensis*, *Echinococcus granulosus*, *Echinococcus multilocularis*, *Enterobius vermicularis*, *Faciola* spp., *Haemonchus* spp., *Heterakis* spp., *Hymenolepis nana, Hyostrongulus* spp., *Loa Loa, Nematodirus* spp., *Oesophagostomum* spp., *Opisthorchis* spp., *Onchocerca volvulus*, *Ostertagia* spp., *Paragonimus* spp., *Schistosomen* spp., *Strongyloides fuelleborni, Strongyloides stercoralis, Stronyloides* spp., *Taenia saginata*, *Taenia solium*, *Trichinella spiralis, Trichinella nativa, Trichinella britovi*, *Trichinella nelsoni*, *Trichinella pseudopsiralis*, *Trichostrongulus* spp., *Trichuris trichuria*, *Wuchereria bancrofti*.

In one embodiment for use in the treatment against ectoparasites, the ectoparasite is one or more insect or arachnid including those of the genera *Ctenocephalides*, *Rhipicephalus*, *Dermacentor, Ixodes, Boophilus*, *Ambylomma, Haemaphysalis*, *Hyalomma, Sarcoptes, Psoroptes, Otodectes, Chorioptes*, *Hypoderma, Damalinia*, *Linognathus*, *Haematopinus*, *Solenoptes, Trichodectes*, and *Felicola.*

In another embodiment for use in the treatment against ectoparasites, the ectoparasite is from the genera *Ctenocephalides*, *Rhipicephalus*, *Dermacentor*, *Ixodes* and/or *Boophilus*. The ectoparasites treated include but are not limited to fleas, ticks, mites, mosquitoes, flies, lice, blowfly and combinations thereof. Specific examples include but are not limited to cat and dog fleas (*Ctenocephalides felis, Ctenocephalides* sp. and the like), ticks (*Rhipicephalus* sp., *Ixodes* sp., *Dermacentor* sp., *Amblyoma* sp. and the like), and mites (*Demodex* sp., *Sarcoptes* sp., *Otodectes* sp. and the like), lice (*Trichodectes* sp., *Cheyletiella* sp., *Lignonathus* sp., and the like), mosquitoes *(Aedes* sp., *Culex* sp., *Anopheles* sp., and the like) and flies (*Hematobia* sp., *Musca* sp., *Stomoxys* sp., *Dematobia* sp., *Cochliomyia* sp., and the like). In yet another embodiment for use in the treatment against ectoparasites, the ectoparasite is a flea and/or tick. Additional examples of ectoparasites include but are not limited to the tick genus *Boophilus*, especially those of the species *microplus* (cattle tick), *decoloratus* and *annulatus*; myiases such as *Dermatobia hominis* (known as Berne in Brazil) and *Cochliomyia hominivorax* (greenbottle); sheep myiases such as *Lucilia sericata, Lucilia cuprina* (known as blowfly strike in Australia, New Zealand and South Africa). Flies proper, namely those whose adult constitutes the parasite, such as *Haematobia irritans* (horn fly); lice such as *Linognathus vitulorum*, etc.; and mites such as *Sarcoptes scabici* and *Psoroptes ovis.* The above list is not exhaustive and other ectoparasites are well known in the art to be harmful to animals and humans. These include, for example migrating dipterous larvae.

When an anthelmintic agent is added to the composition of the invention, the composition can also be for use to treat against endoparasites such as those helminths selected from the group consisting of *Anaplocephala, Ancylostoma, Anecator, Ascaris, Capillaria, Cooperia, Dipylidium, Dirofilaria, Echinococcus*, *Enterobius, Fasciola, Haemonchus, Oesophagostumum*, *Ostertagia, Toxocara, Strongyloides*, *Toxascaris, Trichinella*, *Trichuris*, and *Trichostrongylus*.

In another embodiment of the invention, the compounds and compositions of the invention are suitable for controlling pests such as insects selected from the group consisting of *Blatella germanica*, *Heliothis virescens*, *Leptinotarsa decemlineata, Tetramorium caespitum* and combinations thereof.

The phytoparasitic nematodes include, for example, *Anguina* spp., *Aphelenchoides* spp., *Belonoaimus* spp., *Bursaphelenchus* spp., *Ditylenchus dipsaci, Globodera* spp., *Heliocotylenchus* spp., *Heterodera* spp., *Longidorus* spp., *Meloidogyne* spp., *Pratylenchus* spp., *Radopholus similis, Rotylenchus* spp., *Trichodorus* spp., *Tylenchorhynchus* spp., *Tylenchulus* spp., *Tylenchulus semipenetrans*, *Xiphinema* spp.

In addition, with or without the other pesticidal agents added to the composition, the invention can also be for use to treat other pests which include but are not limited to pests:

(1) from the order of Isopoda, for example *Oniscus asellus*, *Armadillidium vulgare* and *Porcellio scaber;*
(2) from the order of Diplopoda, for example *Blaniulus guttulatus;*
(3) from the order of Chilopoda, for example *Geophilus carpophagus* and *Scutigera* spp.;

(4) from the order of Symphyla, for example *Scutigerella immaculata;*
(5) from the order of Thysanura, for example *Lepisma saccharina;*
(6) from the order of Collembola, for example *Onychiurus armatus;*
(7) from the order of Blattaria, for example *Blatta orientalis, Periplaneta americana, Leucophaea maderae* and *Blattella germanica;*
(8) from the order of Hymenoptera, for example *Diprion* spp., *Hoplocampa* spp., *Lasius* spp., *Monomorium pharaonis* and *Vespa* spp.;
(9) from the order of Siphonaptera, for example *Xenopsylla cheopis* and *Ceratophyllus* spp.;
(10) from the order of Anoplura (Phthiraptera), for example, *Damalinia* spp., *Haematopinus* spp., *Linognathus* spp., *Pediculus* spp., *Trichodectes* spp.;
(11) from the class of Arachnida, for example, *Acarus* siro, *Aceria sheldoni, Aculops* spp., *Aculus* spp., *Amblyomma* spp., *Argas* spp., *Boophilus* spp., *Brevipalpus* spp., *Bryobia praetiosa, Chorioptes* spp., *Dermanyssus gallinae, Eotetranychus* spp., *Epitrimerus pyri, Eutetranychus* spp., *Eriophyes* spp., *Hemitarsonemus* spp., *Hyalomma* spp., *Ixodes* spp., *Latrodectus mactans, Metatetranychus* spp., *Oligonychus* spp., *Ornithodoros* spp., *Panonychus* spp., *Phyllocoptruta oleivora, Polyphagotarsonemus latus, Psoroptes* spp., *Rhipicephalus* spp., *Rhizoglyphus* spp., *Sarcoptes* spp., *Scorpio maurus, Stenotarsonemus* spp., *Tarsonemus* spp., *Tetranychus* spp., *Vasates lycopersici*.;
(12) from the class of Bivalva, for example, *Dreissena* spp.;
(13) from the order of Coleoptera, for example, *Acanthoscelides obtectus, Adoretus* spp., *Agelastica alni, Agriotes* spp., *Amphimallon solstitialis, Anobium punctatum, Anoplophora* spp., *Anthonomus* spp., *Anthrenus* spp., *Apogonia* spp., *Atomaria* spp., *Attagenus* spp., *Bruchidius obtectus, Bruchus* spp., *Ceuthorhynchus* spp., *Cleonus mendicus, Conoderus* spp., *Cosmopolites* spp., *Costelytra zealandica, Curculio* spp., *Cryptorhynchus lapathi, Dermestes* spp., *Diabrotica* spp., *Epilachna* spp., *Faustinus cubae, Gibbium psylloides, Heteronychus arator, Hylamorpha elegans, Hylotrupes bajulus, Hypera postica, Hypothenemus* spp., *Lachnosterna consanguinea, Leptinotarsa decemlineata, Lissorhoptrus oryzophilus, Lixus* spp., *Lyctus* spp., *Meligethes aeneus, Melolontha melolontha, Migdolus* spp., *Monochamus* spp., *Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga* spp., *Popillia japonica, Premnotrypes* spp., *Psylliodes chrysocephala, Ptinus* spp., *Rhizobius ventralis, Rhizopertha dominica, Sitophilus* spp., *Sphenophorus* spp., *Sternechus* spp., *Symphyletes* spp., *Tenebrio molitor, Tribolium* spp., *Trogoderma* spp., *Tychius* spp., *Xylotrechus* spp., *Zabrus* spp.;
(14) from the order of Diptera, for example, *Aedes* spp., *Anopheles* spp., *Bibio hortulanus, Calliphora erythrocephala, Ceratitis capitata, Chrysomyia* spp., *Cochliomyia* spp., *Cordylobia anthropophaga*, *Culex* spp., *Cuterebra* spp., *Dacus oleae, Dermatobia hominis, Drosophila* spp., *Fannia* spp., *Gastrophilus* spp., *Hylemyia* spp., *Hyppobosca* spp., *Hypoderma* spp., *Liriomyza* spp., *Lucilia* spp., *Musca* spp., *Nezara* spp., *Oestrus* spp., *Oscinella frit, Pegomyia hyoscyami, Phorbia* spp., *Stomoxys* spp., *Tabanus* spp., *Tannia* spp., *Tipula paludosa, Wohlfahrtia* spp.;
(15) from the class of Gastropoda, for example, *Arion* spp., *Biomphalaria* spp., *Bulinus* spp., *Deroceras* spp., *Galba* spp., *Lymnaea* spp., *Oncomelania* spp., *Succinea* spp.;
(16) from the class of helminths, for example, *Ancylostoma duodenale, Ancylostoma ceylanicum, Acylostoma braziliensis*, *Ancylostoma* spp., *Ascaris lubricoides*, *Ascaris* spp., *Brugia malayi, Brugia timori, Bunostomum* spp., *Chabertia* spp., *Clonorchis* spp., *Cooperia* spp., *Dicrocoelium* spp, *Dictyocaulus filaria, Diphyllobothrium latum, Dracunculus medinensis, Echinococcus granulosus, Echinococcus multilocularis, Enterobius vermicularis, Faciola* spp., *Haemonchus* spp., *Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni, Strongyloides stercoralis*, *Stronyloides* spp., *Taenia saginata, Taenia solium, Trichinella spiralis*, *Trichinella nativa, Trichinella britovi*, *Trichinella nelsoni, Trichinella pseudopsiralis*, *Trichostrongulus* spp., *Trichuris trichuria*, *Wuchereria bancrofti*.;
(17) from the order of Heteroptera, for example, *Anasa tristis*, *Antestiopsis* spp., *Blissus* spp., *Calocoris* spp., *Campylomma livida, Cavelerius* spp., *Cimex* spp., *Creontiades dilutus, Dasynus piperis*, *Dichelops furcatus, Diconocoris hewetti, Dysdercus* spp., *Euschistus* spp., *Eurygaster* spp., *Heliopeltis* spp., *Horcias nobilellus, Leptocorisa* spp., *Leptoglossus phyllopus, Lygus* spp., *Macropes excavatus, Miridae*, *Nezara* spp., *Oebalus* spp., *Pentomidae, Piesma quadrata, Piezodorus* spp., *Psallus seriatus*, *Pseudacysta persea*, *Rhodnius* spp., *Sahlbergella singularis*, *Scotinophora* spp., *Stephanitis nashi, Tibraca* spp., *Triatoma* spp.;
(18) from the order of Homoptera, for example, *Acyrthosipon* spp., *Aeneolamia* spp., *Agonoscena* spp., *Aleurodes* spp., *Aleurolobus barodensis*, *Aleurothrixus* spp., *Amrasca* spp., *Anuraphis cardui*, *Aonidiella* spp., *Aphanostigma piri*, *Aphis* spp., *Arboridia apicalis, Aspidiella* spp., *Aspidiotus* spp., *Atanus* spp., *Aulacorthum solani, Bemisia* spp., *Brachycaudus helichrysii, Brachycolus* spp., *Brevicoryne brassicae*, *Calligypona marginata, Carneocephala fulgida, Ceratovacuna lanigera*, *Cercopidae*, *Ceroplastes* spp., *Chaetosiphon fragaefolii, Chionaspis tegalensis, Chlorita onukii, Chromaphis juglandicola, Chrysomphalus ficus, Cicadulina mbila, Coccomytilus halli, Coccus* spp., *Cryptomyzus ribis*, *Dalbulus* spp., *Dialeurodes* spp., *Diaphorina* spp., *Diaspis* spp., *Doralis* spp., *Drosicha* spp., *Dysaphis* spp., *Dysmicoccus* spp., *Empoasca* spp., *Eriosoma* spp., *Erythroneura* spp., *Euscelis bilobatus, Geococcus coffeae, Homalodisca coagulata, Hyalopterus arundinis, Icerya* spp., *Idiocerus* spp., *Idioscopus* spp., *Laodelphax striatellus, Lecanium* spp., *Lepidosaphes* spp., *Lipaphis erysimi, Macrosiphum* spp., *Mahanarva fimbriolata, Melanaphis sacchari*, *Metcalfiella* spp., *Metopolophium dirhodum, Monellia costalis, Monelliopsis pecanis, Myzus* spp., *Nasonovia ribisnigri*, *Nephotettix* spp., *Nilaparvata lugens, Oncometopia* spp., *Orthezia praelonga*, *Parabemisia myricae, Paratrioza* spp., *Parlatoria* spp., *Pemphigus* spp., *Peregrinus maidis, Phenacoccus* spp., *Phloeomyzus passerinii*, *Phorodon humuli, Phylloxera* spp., *Pinnaspis aspidistrae*, *Planococcus* spp., *Protopulvinaria pyriformis, Pseudaulacaspis pentagona, Pseudococcus* spp., *Psylla* spp., *Pteromalus* spp., *Pyrilla* spp., *Quadraspidiotus* spp., *Quesada gigas, Rastrococcus* spp., *Rhopalosiphum* spp., *Saissetia* spp., *Scaphoides titanus, Schizaphis graminum, Selenaspidus articulatus, Sogata* spp., *Sogatella furcifera, Sogatodes* spp., *Stictocephala festina, Tenalaphara malayensis, Tinocallis caryaefoliae, Tomaspis* spp., *Toxoptera* spp., *Trialeurodes vaporariorum, Trioza* spp., *Typhlocyba* spp., *Unaspis* spp., *Viteus vitifolii*.;

(19) from the order of Isoptera, for example, *Reticulitermes* spp., *Odontotermes* spp.;

(20) from the order of Lepidoptera, for example, *Acronicta major, Aedia leucomelas, Agrotis* spp., *Alabama argillacea*, *Anticarsia* spp., *Barathra brassicae*, *Bucculatrix thurberiella, Bupalus piniarius, Cacoecia podana, Capua reticulana, Carpocapsa pomonella, Cheimatobia brumata, Chilo* spp., *Choristoneura fumiferana, Clysia ambiguella, Cnaphalocerus* spp., *Earias insulana, Ephestia kuehniella, Euproctis chrysorrhoea, Euxoa* spp., *Feltia* spp., *Galleria mellonella, Helicoverpa* spp., *Heliothis* spp., *Hofmannophila pseudospretella, Homona magnanima, Hyponomeuta padella, Laphygma* spp., *Lithocolletis blancardella*, *Lithophane antennata, Loxagrotis albicosta, Lymantria* spp., *Malacosoma neustria, Mamestra brassicae*, *Mocis repanda*, *Mythimna separate, Oria* spp., *Oulema oryzae, Panolis flammea, Pectinophora gossypiella, Phyllocnistis citrella*, *Pieris* spp., *Plutella xylostella, Prodenia* spp., *Pseudaletia* spp., *Pseudoplusia includens, Pyrausta nubilalis, Spodoptera* spp., *Thermesia gemmatalis, Tinea pellionella, Tineola bisselliella, Tortrix viridana*, *Trichoplusia* spp.;

(21) from the order of Orthoptera, for example, *Acheta domesticus, Blatta orientals, Blattella germanica*, *Gryllotalpa* spp., *Leucophaea maderae*, *Locusta* spp., *Melanoplus* spp., *Periplaneta americana*, *Schistocerca gregaria.;*

(22) from the order of Thysanoptera, for example, *Baliothrips biformis*, *Enneothrips flavens, Frankliniella* spp., *Heliothrips* spp., *Hercinothrips femoralis, Kakothrips* spp., *Rhipiphorothrips cruentatus*, *Scirtothrips* spp., *Taeniothrips cardamoni, Thrips* spp.;

(23) from the class of Protozoa, for example, *Eimeria* spp.

## EXAMPLES

The following examples are provided to illustrate certain embodiments of the invention and are not to be construed in any way as limiting the scope of the invention.

Example 1 is particularly, though not exclusively, representative of injectable eprinomectin / levamisole formulations according to the present invention. Example 2 provides evidence that ivermectin-containing formulations according to the present invention are stable and safe in animals. Finally, example 3 provides extensive, though not sole evidence of the significant amount of time and effort that was invested in developing the inventive formulations.

<u>Example 1 - Development of stable injectable levamisole / eprinomectin formulations</u>

Levamisole is water soluble and eprinomectin is nearly water insoluble. Eprinomectin is soluble, for example, in methanol, ethanol, ethyl acetate, and dimethyl acetate (DMA). Applicants are aware of no existing combination injectable formulation comprising eprinomectin and levamisole. The development work fully disclosed and described herein out was carried out to provide a formulation in which the anthelmintics eprinomectin and levamisole (as the phosphate) could be combined in a formulation having physical and chemical properties suitable for injection.

Stress studies have identified at least two formulations that are stable and suitable in terms of irritancy.

Constituents of these formulations are provided in Table 1.

**Table 1:** Formulation of Eprinomectin levamisole Injection

| Ingredient | Formulation 1 | Formulation 2 | Function |
|---|---|---|---|
| | % w/v Composition | % w/v Composition | |
| Eprinomectin | 0.70* | 0.70* | Active |
| Levamisole Phosphate | 22.3* | 22.3* | Active |
| BHT | 0.1 | 0.1 | Antioxidant |

(continued)

| Ingredient | Formulation 1 | Formulation 2 | Function |
|---|---|---|---|
| | % w/v Composition | % w/v Composition | |
| Dimethylacetamide | 5.0 | 5.0 | Solvent |
| Methyl Paraben | 0.15 | 0.15 | Preservative |
| Polysorbate 80 | 5.0 | - | Surfactant |
| Cremophor EL | - | 5.0 | Surfactant |
| Water For Injection | q.s. to 100% | | Vehicle |
| *Corrected for potency | | | |

Stability of both formulations has been demonstrated in real-time storage conditions.

**Table 2:** Stability of Eprinomectin Levamisole Injection Formulation 1

| Batch No. | | 2-8°C | 25°C / 60% RH | | |
|---|---|---|---|---|---|
| | | ***Control*** | ***1 month*** | ***2 month*** | ***3 month*** |
| Formulation 1. | Description | VLYS | VLYS | VLYS | VLYS |
| | Assay of Eprinomectin | 100.9 | 99.6 | 98.0 | 98.6 |
| | Assay of Levamisole Phosphate | 98.0 | 97.9 | 98.3 | 99.2 |
| VLYS: Very Light Yellow Solution<br>LYS: Light Yellow Solution<br>YS: Yellow Solution | | | | | |

**Table 3:** Stability of Eprinomectin Levamisole Injection Formulation 2

| Batch No. | | 2-8°C | 25°C / 60% RH | | |
|---|---|---|---|---|---|
| | | ***Control*** | ***1 month*** | ***2 month*** | ***3 month*** |
| Formulation 2. | Description | VLYS | VLYS | VLYS | VLYS |
| | Assay of Eprinomectin | 101.1 | 100.0 | 98.0 | 99.6 |
| | Assay of Levamisole Phosphate | 98.9 | 99.3 | 98.8 | 100.3 |
| VLYS: Very Light Yellow Solution<br>LYS: Light Yellow Solution<br>YS: Yellow Solution | | | | | |

**Table 4:** Administration of Treatments

Both formulations were also tested in cattle studies to determine the absorption characteristics and injection site acceptability. Twenty-one Hereford Cross 15 month old female cattle were selected for the study from a larger group held at the study site. On Day -1 the study animals were individually identified with uniquely numbered ear tags.

The study animals were weighed and ranked according to body weight. The animal with the median body weight (305 kg) was selected as the control animal. The remaining animals were assigned to two groups of ten by restrictive randomization based on body weight. Treatments were administered as per Table 4.

Animals in Group 1 were treated with Formulation 1 at a dose rate of 1ml/35kg body

| Animal ID | Group 1D | Product/ BN | Weight (kg) | Dose (ml) |
|---|---|---|---|---|
| 2 | 1 | Formulation 1 BN:08 | 324.0 | 9.3 |
| 3 | | | 294.0 | 8.4 |
| 5 | | | 334.0 | 9.5 |
| 6 | | | 315.0 | 9.0 |
| 9 | | | 343.0 | 9.8 |
| 11 | | | 297.0 | 8.5 |
| 12 | | | 285.0 | 8.1 |
| 13 | | | 289.0 | 8.3 |
| 14 | | | 302.0 | 8.6 |
| 20 | | | 313.0 | 8.9 |
| | | | | |
| 1 | 2 | Formulation 2 BN:09 | 270.0 | 7.7 |
| 4 | | | 303.0 | 8.7 |
| 7 | | | 288.0 | 8.2 |
| 8 | | | 317.0 | 9.1 |
| 10 | | | 326.0 | 9.3 |
| 16 | | | 331.0 | 9.5 |
| 17 | | | 291.0 | 8.3 |
| 18 | | | 309.0 | 8.8 |
| 19 | | | 296.0 | 8.5 |
| 21 | | | 340.0 | 9.7 |
| 15 | 3 | Control | 305.0 | ------ |
| weight. Animals in Group 2 were treated with Formulation 2 at a dose rate of 1ml/35kg bodyweight. All calculated doses were rounded up to the nearest 0.2ml. The control animal received no treatment. | | | | |

Treatments were administered by subcutaneous injection to the anterior half of the neck, using a new sterile 18 gauge 25mm needle for each injection. The area for injection was clipped and swabbed with 70% isopropyl alcohol prior to injection. The injection sites were inspected and palpated at 24, 48 and 96 hours post treatment and on Days 10, 21 and 35. All observations were recorded.

The degree of injection site swelling was recorded according to the following scale:

0 = no edema
1 = very slight edema (barely noticed)
2 = slight edema (well defined by definite raising)
3 = moderate edema (raised approximately 1mm)
4 = (raised more than 1 mm and extending beyond the area of injection)

The maximum length, width and height of any visible or palpable site reaction was determined using calipers, and all findings were recorded.

**Table 5:** Injection Site Reactions Formulation 1

| Formulation 1 Batch no:08 | | | | | | | | |
|---|---|---|---|---|---|---|---|---|
| **Animal ID** | **Weight (kg)** | **Dose (mL)** | **24 hours** | **48 hours** | **96 hours** | **Day 10** | **Day 21** | **Day 35** |
| 2 | 324.0 | 9.3 | 4 | 4 | 4 | 4 | 1 | 0 |
| 3 | 294.0 | 8.4 | 4 | 2 | 1 | 0 | 0 | 0 |
| 5 | 334.0 | 9.5 | 4 | 1 | 1 | 0 | 0 | 0 |
| 6 | 315.0 | 9.0 | 4 | 4 | 4 | 4 | 3 | 1 |
| 9 | 343.0 | 9.8 | 0 | 0 | 1 | 0 | 0 | 0 |
| 11 | 297.0 | 8.5 | 4 | 3 | 1 | 1 | 0 | 0 |
| 12 | 285.0 | 8.1 | 4 | 2 | 1 | 1 | 0 | 0 |
| 13 | 289.0 | 8.3 | 4 | 4 | 3 | 2 | 0 | 0 |
| 14 | 302.0 | 8.6 | 4 | 4 | 1 | 1 | 0 | 0 |
| 20 | 313.0 | 8.9 | 4 | 4 | 1 | 0 | 0 | 0 |

**Table 6:** Injection Site Reactions Formulation 2

| Formulation 2 Batch no:09 | | | | | | | | |
|---|---|---|---|---|---|---|---|---|
| **Animal ID** | **Weight (kg)** | **Dose (mL)** | **24 hours** | **48 hours** | **96 hours** | **Day 10** | **Day 21** | **Day 35** |
| 1 | 270.0 | 7.7 | 4 | 4 | 3 | 1 | 0 | 0 |
| 4 | 303.0 | 8.7 | 4 | 4 | 1 | 0 | 0 | 0 |
| 7 | 288.0 | 8.2 | 4 | 4 | 4 | 0 | 0 | 0 |
| 8 | 317.0 | 9.1 | 4 | 4 | 4 | 3 | 0 | 0 |
| 10 | 326.0 | 9.3 | 4 | 4 | 0 | 0 | 0 | 0 |
| 16 | 331.0 | 9.5 | 4 | 1 | 0 | 0 | 0 | 0 |
| 17 | 291.0 | 8.3 | 4 | 4 | 4 | 1 | 0 | 0 |
| 18 | 309.0 | 8.8 | 4 | 3 | 1 | 0 | 0 | 0 |
| 19 | 296.0 | 8.5 | 1 | 1 | 1 | 0 | 0 | 0 |
| 21 | 340.0 | 9.7 | 4 | 4 | 1 | 1 | 0 | 0 |

**Blood plasma profile**

On Day -1, (3 Jan 2010) five animals were randomly selected from each of the two treated groups for blood sampling as illustrated in Table 7:

**Table 7:** Eprinomectin Levamisole Blood Sampling Schedule

| Blood Plasma Group 1 | Blood Plasma Group 2 |
|---|---|
| 3 | 1 |
| 11 | 10 |
| 12 | 16 |
| 14 | 18 |
| 20 | 21 |

These ten animals plus the untreated control were blood sampled via jugular venipuncture on Day -1 prior to treatment. These same animals were blood sampled at 1, 2, 6, 12, 24, 48 and 96 hours post treatment, and then at Day 10, Day 21 and Day 35 after treatment. Blood samples were collected by caudal venipuncture into green top heparinised Vacutainer's, and spun in a centrifuge for ten minutes at 3000 RPM. Plasma for each animal was decanted into individually labeled plasma vials and frozen within two hours of collection.

At the conclusion of the animal phase of the study (Day 35) the frozen plasma samples were submitted to an analytical laboratory for Eprinomectin and Levamisole analysis. Samples from 0, 1, 2, 6, 12, 24 and 48 hours were analyzed for Levamisole concentration. Samples from 0, 48, 96 hours and 10, 20 and 35 days were analyzed for Eprinomectin concentration.

The mean eprinomectin and levamisole pharmacokinetic profiles in cattle plasma after treatment with the two formulations are presented in the figures below. Results demonstrate that both formulations resulted in almost identical plasma profiles for both levamisole and eprinomectin. FIGs. 2 and 3 summarize the data.

Table 8: Excipients used in the Eprinomectin / Levamisole injectable formulations

| Ingredient | CAS No. | Formulation 1 | Formulation 2 | Function |
|---|---|---|---|---|
| | | % w/v Composition | % w/v Composition | |
| BHT | 128-37-0 | 0.1 | 0.1 | Antioxidant |
| Dimethylacetamide | 127-19-5 | 5.0 | 5.0 | Solvent |
| Methyl Paraben | 99-76-3 | 0.15 | 0.15 | Preservative |
| Polysorbate 80 | 9005-65-6 | 5.0 | - | Surfactant |
| Cremophor EL | 61796-12-6 | - | 5.0 | Surfactant |
| Water For Injection | 7789-20-0 | q.s. to 100% | q.s. to 100% | Vehicle |

Formulation Development

A number of solvents were evaluated including Glycerol Formal, 2-Pyrol, Propylene Glycol, Miglyol 840 and DMA from 2 - 20% (see Example 2). The preferred batches contained a surfactant such as Cremophor EL, Polysorbate 80 and modified Lecithin in the range 2 - 5%. Vehicles trialed included Water, Glycerol Formal and modified vegetable oil. The combination of 5% DMA and 5% CREMAPHOR EL or POLYSORBATE 80 was demonstrated to be quite useful for the levamisole / ivermectin formulations according to the present invention.

Formulations comprising eprinomectin instead of ivermectin were evaluated using CREMAPHOR EL and several other surfactants. The stability data provided above indicated that eprinomectin performed at least as well as ivermectin. A series of batches were prepared comparing the addition of BHT as an antioxidant to batches without antioxidant. Stress studies showed a significant improvement in stability of the actives when BHT was added to the formulation. Finally, Methyl Paraben was selected for a preservative as the marketed product may be provided as a multi-dose pack.

Physicochemical Properties

Assessment of developmental formulation batches was generally performed on the basis of appearance, including color, of the solution and the assay content of both of the active ingredients. The description of appearance for the proposed formulation is that of a clear light yellow to yellow solution. The physicochemical properties investigated during the stress study monitored the assay of the two actives. Density and pH values were also monitored. Density was in the range 1.05 - 1.10 and pH in the range 3.0 - 5.0. Visual assessment shows the product to be flowable with a water-like appearance.

The bulk of the WFI was added to the main manufacturing vessel and methyl paraben, the water soluble preservative, was added first to maintain microbial resistance. Levamisole Phosphate was added next as it water soluble and at a relatively high concentration. In a separate vessel, eprinomectin and BHT were dissolved in DMA and the surfactant added next. This combination was then added to the bulk aqueous phase with mixing. Eprinomectin is not water soluble, so this carried the eprinomectin into and maintained it in solution. Finally the batch was made to volume with WFI and mixed. All mixing was accomplished using simple mechanical agitation. Heat was only required to dissolve the Methyl Paraben, and once all soluble components were dissolved, the WFI was cooled before further processing occurs.

Example 2

Preliminary work focused on stability of ivermectin / levamisole-containing formulations, though subsequent studies (summarized in Example 1) shifted to the related macrocyclic lactone eprinomectin. As mentioned, eprinomectin offers a significant advantage in terms of short withdrawal time due its relatively low milk to plasma partition ratio. Table 9 summarizes batches 2-13.

Table 9. Formulation summary: IVM is ivermectin and LEV is levamisole phosphate

| Batch No | Batch size | Formulation | | Source and batch number of APIs | | Purpose, method of manufacture and comments |
|---|---|---|---|---|---|---|
| | | | % w/v | IVM | LEV | |
| 02 (REF) | 100mL | Ivermectin<br>Levamisole $PO_4$<br>Glycerol Formal<br>Benzyl Alcohol<br>Polysorbate 80<br>WFI | 0.70<br>22.3<br>20.0<br>1.0<br>5.0<br>to 100 | Hisun 080340 | Guilin 020903 | Purpose: Preliminary screening trial to determine a suitable base for Ivermectin and Levamisole Phosphate |
| 03 | 100mL | Ivermectin<br>Levamisole $PO_4$<br>DMA<br>Cremophor EL<br>WFI | 0.70<br>22.3<br>2.0<br>2.0<br>to 100 | Hisun 080340 | Guilin 020903 | (see stability table below) |
| 04 (REF) | 100mL | Ivermectin<br>Levamisole $PO_4$<br>Propylene Glycol<br>Benzyl Alcohol<br>WFI | 0.70<br>22.3<br>20.0<br>1.5<br>to 100 | Hisun 080340 | Guilin 020903 | |
| 06 (REF) | 100mL | Ivermectin<br>Levamisole $PO_4$<br>Miglyol 840<br>Phospholipon<br>Glycerol Formal<br>WFI | 0.70<br>22.3<br>20.0<br>1.8<br>2.2<br>to 100 | Hisun 080340 | Guilin 090601 | Result: Batch 03 (07) batch 06 most suitable formulations. Batch 03, a solution preferred over batch 06 an emulsion. |
| 07 (Repeat 03) | 100mL | Ivermectin<br>Levamisole $PO_4$<br>DMA<br>Cremophor EL<br>WFI | 0.70<br>22.3<br>2.0<br>2.0<br>to 100 | Hisun 070403 | Guilin 090601 | |

| | 55°C 5 days<br>Assay compared to initial | |
|---|---|---|
| | IVM | LEV |
| 02 | 78.0% | 94.4% |
| 03 | 101.2% | 94.1% |
| 04 | 96.5% | 86.3% |
| 06 | 99.6% | 99.7% |
| 07 | 98.0% | 94.5% |
| 08 | 125.3% | 96.1% |

(continued)

<table>
<tr><th>Batch No</th><th>Batch size</th><th colspan="2">Formulation</th><th colspan="2">Source and batch number of APIs</th><th>Purpose, method of manufacture and comments</th></tr>
<tr><td></td><td></td><td></td><td>% w/v</td><td>IVM</td><td>LEV</td><td></td></tr>
<tr><td>08 (REF)</td><td>100mL</td><td>Ivermectin<br>Levamisole $PO_4$<br>WFI<br>Montanide</td><td>0.70<br>22.3<br>30.0<br>to 100</td><td>Hisun 070403</td><td>Guilin 090601</td><td></td></tr>
<tr><td>09</td><td>100mL</td><td>Ivermectin<br>Levamisole $PO_4$<br>DMA<br>Cremophor EL<br>WFI</td><td>0.70<br>22.3<br>2.0<br>2.0<br>to 100</td><td>Hisun 080340</td><td>Guilin 090601</td><td rowspan="2">Purpose: Repeat batch 03 using two different batches of Ivermectin<br><table><tr><th></th><th colspan="2">55°C 5 days</th></tr><tr><th></th><th colspan="2">Percent initial</th></tr><tr><th></th><th>IVM</th><th>LEV</th></tr><tr><td>09</td><td>83.6%</td><td>97.9%</td></tr><tr><td>10</td><td>58.4%</td><td>97.6%</td></tr></table><br>Result: Crystallization observed.</td></tr>
<tr><td>10</td><td>100mL</td><td>Ivermectin<br>Levamisole $PO_4$<br>DMA<br>Cremophor EL<br>WFI</td><td>0.70<br>22.3<br>2.0<br>2.0<br>to 100</td><td>Hisun 070403</td><td>Guilin 090601</td></tr>
<tr><td>11</td><td>100mL</td><td>Ivermectin<br>Levamisole $PO_4$<br>DMA<br>Cremophor EL<br>WFI</td><td>0.70<br>22.3<br>5.0<br>5.0<br>to 100</td><td>Hisun 080340</td><td>Guilin 090601</td><td>Purpose: Repeat batch 03 but increase surfactant and solvent levels. Use two different batches of Ivermectin.</td></tr>
<tr><td>12</td><td>100mL</td><td>Ivermectin<br>Levamisole $PO_4$<br>DMA<br>Cremophor EL<br>WFI</td><td>0.70<br>22.3<br>5.0<br>5.0<br>to 100</td><td>Hisun 070403</td><td>Guilin 090601</td><td></td></tr>
</table>

(continued)

<table>
<tr><th>Batch No</th><th>Batch size</th><th colspan="2">Formulation</th><th colspan="2">Source and batch number of APIs</th><th>Purpose, method of manufacture and comments</th></tr>
<tr><td></td><td></td><td></td><td>% w/v</td><td>IVM</td><td>LEV</td><td></td></tr>
<tr><td>13</td><td>300mL</td><td>Ivermectin<br>Levamisole $PO_4$<br>DMA<br>Cremophor EL<br>WFI</td><td>0.70<br>22.3<br>5.0<br>5.0<br>to 100</td><td>Hisun 070403</td><td>Guilin 090601</td><td>Purpose: Repeat batch 12 with stress testing at 40°C and 55°C.<br><table><tr><td></td><td colspan="2">5 days</td></tr><tr><td></td><td colspan="2">Percent initial</td></tr><tr><td></td><td>IVM</td><td>LEV</td></tr><tr><td>40° C</td><td>98.3%</td><td>99.6%</td></tr><tr><td>55° C</td><td>85.2%</td><td>97.3%</td></tr></table>Result: Stability improved.</td></tr>
</table>

Example 3 (Reference) -Alternative Formulations:

The following example illustrates the significant time and effort that was invested in developing the stable, injectable formulations according to the present invention. A person of ordinary skill will immediately appreciate the rigorous experimentation that was, and is very often required in order to arrive at the proper blend of actives and excipients to produce an effective, pharmaceutically acceptable formulation. Injectable formulations tend to be particularly challenging to develop, as factors such as excessive viscosity and injection site irritation can preclude the use of otherwise effective excipients (e.g. excipients which provide good stability for the combination of active pharmaceutical ingredients).

Pre-formulation development efforts commenced using the hydrochloride salt of Levamisole. Preliminary solubility data strongly contra-indicated the use of NMP and Glycerin as a single solvent system to solubilize Levamisole and the use of vegetable oil to solubilize the Ivermectin. A large number of surfactants and stabilizers including CAPMUL MCM, TWEEN 80, SPAN 20, CREMAPHOR RH 40, PVP K-30 and SODIUM CMC were trialed. Also trialed were several buffers, including phosphate and acetate salts before citrate was finally selected based upon the desirability / stability of the resulting formulations.

Early indications were that CAPMUL MCM (medium chain mono and diglycerides) aqueous based formulations might provide a clinically acceptable base for injection. Batches INJ0003-01 to INJ0003-11 were therefore formulated with Levamisole using either the hydrochloride or the phosphate salts thereof (formulation compositions are summarized in Table 10). CAPMUL MCM and TWEEN 80 with a citrate buffer was the basis of this aqueous formulation. Although considered stable at the planned storage of 2 - 8°C, unacceptable site reactions occurred during clinical investigation.

**Table 10:** Formulation summary - batches INJ003-01 through INJ003-11

<table>
<tr><th>Batch No</th><th>Batch size</th><th colspan="2">Formulation</th><th>Purpose, method of manufacture and comments</th></tr>
<tr><td></td><td></td><td></td><td>% w/v</td><td></td></tr>
<tr><td>INJ003-01</td><td>400mL</td><td>Ivermectin<br>Levamisole HCl<br>Capmul MCM<br>Tween 80<br>Tri Sodium Citrate<br>Conc. HCl</td><td>0.48<br>20.0<br>25.0<br>15.0<br>1.5<br>0.25 mL</td><td>Purpose: Preliminary screening trial to determine a suitable base for Ivermectin and Levamisole HCl<br><table><tr><td></td><td>25°C</td></tr><tr><td></td><td>Assay compared to initial</td></tr></table></td></tr>
</table>

(continued)

| Batch No | Batch size | Formulation | | Purpose, method of manufacture and comments |
|---|---|---|---|---|
| | | | % w/v | |
| | | WFI | to 100 | |

| | Ivermectin | Levamisole HCl |
|---|---|---|
| 1 M | 99% | 98% |
| 2 M | 98% | 99% |
| 4 M | 97% | 97% |
| 6 M | 95% | 98% |
| 9 M | 93% | 97% |
| 12 M | 92% | 97% |

| | 40°C | |
|---|---|---|
| | Assay compared to initial | |
| | **Ivermectin** | **Levamisole HCl** |
| 1 M | 95% | 95% |
| 2 M | 91% | 96% |
| 4 M | 86% | 92% |
| 6 M | 78% | 90% |

| | 55°C | |
|---|---|---|
| | Assay compared to initial | |
| | **Ivermectin** | **Levamisole HCl** |
| 2 wk | 84% | 95% |
| 4 wk | 70% | 93% |

(continued)

<table>
<tr><th rowspan="2">Batch No</th><th rowspan="2">Batch size</th><th colspan="2">Formulation</th><th rowspan="2">Purpose, method of manufacture and comments</th></tr>
<tr><th></th><th>% w/v</th></tr>
<tr><td>INJ003 -02</td><td>400mL</td><td>Ivermectin<br>Levamisole HCl<br>Capmul MCM<br>Tween 80<br>Tri Sodium Citrate<br>Cone. HCl<br>WFI</td><td>0.48<br>20.0<br>10.0<br>10.0<br>2.0<br>0.25 mL<br>to 100</td><td>Purpose: Preliminary screening trial to determine a suitable base for Ivermectin and Levamisole HCl
<table>
<tr><th></th><th colspan="2">25°C</th></tr>
<tr><th></th><th colspan="2">Assay compared to initial</th></tr>
<tr><th></th><th>Ivermectin</th><th>Levamisole HCl</th></tr>
<tr><td>1 M</td><td>97%</td><td>97%</td></tr>
<tr><td>2 M</td><td>95%</td><td>96%</td></tr>
<tr><td>4 M</td><td>93%</td><td>97%</td></tr>
<tr><td>6 M</td><td>91%</td><td>95%</td></tr>
<tr><td>9 M</td><td>90%</td><td>96%</td></tr>
<tr><td>12 M</td><td>87%</td><td>96%</td></tr>
</table>
<table>
<tr><th></th><th colspan="2">40°C</th></tr>
<tr><th></th><th colspan="2">Assay compared to initial</th></tr>
<tr><th></th><th>Ivermectin</th><th>Levamisole HCl</th></tr>
<tr><td>1 M</td><td>90%</td><td>94%</td></tr>
<tr><td>2 M</td><td>83%</td><td>91%</td></tr>
<tr><td>4 M</td><td>79%</td><td>91%</td></tr>
<tr><td>6 M</td><td>75%</td><td>88%</td></tr>
</table>
<table>
<tr><th></th><th colspan="2">55°C</th></tr>
<tr><th></th><th colspan="2">Assay compared to initial</th></tr>
<tr><th></th><th>Ivermectin</th><th>Levamisole HCl</th></tr>
<tr><td>2 wk</td><td>74%</td><td>89%</td></tr>
<tr><td>4 wk</td><td>69%</td><td>83%</td></tr>
</table></td></tr>
<tr><td>INJ003 -03</td><td>1000mL</td><td>Ivermectin<br>Levamisole HCl<br>Capmul MCM<br>Tween 80<br>Tri Sodium Citrate<br>Conc. HCl</td><td>0.48<br>16.11<br>25.0<br>15.0<br>1.5<br>0.25 mL</td><td>Purpose: Batch submit for site reaction study
<table>
<tr><th></th><th colspan="2">25°C</th></tr>
<tr><th></th><th colspan="2">Assay compared to initial</th></tr>
<tr><th></th><th>Ivermectin</th><th>Levamisole HCl</th></tr>
</table></td></tr>
</table>

(continued)

| Batch No | Batch size | Formulation | % w/v | Purpose, method of manufacture and comments | | | |
|---|---|---|---|---|---|---|---|
| | | WFI | to 100 | | 1 M | 98% | 99% |
| | | | | | 2 M | 94% | 96% |
| | | | | | 4 M | 94% | 99% |
| INJ003-04 | 500 mL | Ivermectin | 0.48 | Purpose: Same formula as batch INJ003-01 | | | |
| | | Levamisole HCl | 20.0 | | | | |
| | | Capmul MCM | 25.0 | | | | |
| | | Tween 80 | 15.0 | | | 25°C | |
| | | Tri Sodium Citrate | 1.5 | | | Assay compared to initial | |
| | | Conc. HCl | 0.25 mL | | | **Ivermectin** | **Levamisole HCl** |
| | | WFI | to 100 | | 1 M | 97% | 100% |
| | | | | | 2 M | 97% | 99% |
| | | | | | 4 M | 96% | 98% |
| INJ003-05 | 500 mL | Ivermectin | 0.48 | Purpose: Same formula as batch INJ003-01 | | | |
| | | Levamisole HCl | 20.0 | | | | |
| | | Capmul MCM | 25.0 | | | | |
| | | Tween 80 | 15.0 | | | 25°C | |
| | | Tri Sodium Citrate | 1.5 | | | Assay compared to initial | |
| | | Conc. HCl | 0.25 mL | | | **Ivermectin** | **Levamisole HCl** |
| | | WFI | to 100 | | 1 M | 89% | 79% |
| | | | | | 2 M | 92% | 81% |
| | | | | | 4 M | 87% | 77% |
| INJ003-06 | 500 mL | Ivermectin | 0.48 | Purpose: Same formula as batch INJ003-01 - with parabens | | | |
| | | Levamisole HCl | 20.0 | | | | |
| | | Capmul MCM | 25.0 | | | | |
| | | Tween 80 | 15.0 | | | | |
| | | Tri Sodium Citrate | 1.5 | | | 25°C | |
| | | Methylparaben | 0.18 | | | Assay compared to initial | |
| | | Propylparaben | 0.20 | | | **Ivermectin** | **Levamisole HCl** |
| | | Conc. HCl | 0.25 mL | | 1 M | 99% | 99% |
| | | WFI | to 100 | | | | |
| | | | | | 2 M | 100% | 98% |
| | | | | | 4 M | 96% | 99% |

(continued)

| Batch No | Batch size | Formulation | % w/v | Purpose, method of manufacture and comments |
|---|---|---|---|---|
| | | | | |
| INJ003-07 | 1500 mL | Ivermectin<br>Levamisole $PO_4$<br>Capmul MCM<br>Tween 80<br>BHT<br>Methylparaben<br>Propylparaben<br>Tri Sodium Citrate<br>Propyl gallate<br>Conc. HCl<br>WFI | 0.57<br>20.0<br>25.0<br>15.0<br>0.02<br>0.18<br>0.20<br>1.5<br>0.02<br>0.25 mL<br>to 100 | Purpose: Batch with Levamisole $PO_4$ & BHT<br><br>Results: Sample kept at 55°C were separated into two layers in three weeks |
| INJ003-08 | 1500 mL | Ivermectin<br>Levamisole $PO_4$<br>Capmul MCM<br>Tween 80<br>Methylparaben<br>Propylparaben<br>Tri Sodium Citrate<br>Propyl gallate<br>Conc. HCl<br>WFI | 0.57<br>20.0<br>25.0<br>15.0<br>0.18<br>0.20<br>1.5<br>0.02<br>0.25 mL<br>to 100 | Purpose: Batch with Levamisole $PO_4$ & nil BHT<br><br>Results: Sample kept at 55°C were separated into two layers in three weeks |
| INJ003-09 | 1500 mL | Ivermectin<br>Levamisole $PO_4$<br>Capmul MCM<br>Tween 80<br>BHT<br>Methylparaben<br>Propylparaben<br>Tri Sodium Citrate<br>Propyl gallate<br>Conc. HCl | 0.57<br>20.0<br>25.0<br>15.0<br>0.03<br>0.18<br>0.20<br>1.5<br>0.02<br>0.25 mL | Purpose: Batch with Levamisole $PO_4$ & BHT with pH 4.5 |
| | | WFI | to 100 | |

| | 2-8°C | |
|---|---|---|
| | Assay compared to initial | |
| | **Ivermectin** | **Levamisole $PO_4$** |
| 1 M | 103 | 104 |
| 2 M | 105 | 100 |
| 3 M | 100 | 99 |
| 6 M | 103 | 101 |
| 9 M | 101 | 99 |

(continued)

<table>
<tr><th rowspan="2">Batch No</th><th rowspan="2">Batch size</th><th colspan="2">Formulation</th><th rowspan="2">Purpose, method of manufacture and comments</th></tr>
<tr><th></th><th>% w/v</th></tr>
<tr><td></td><td></td><td></td><td></td><td>
<table>
<tr><th rowspan="3"></th><th colspan="2">25°C</th></tr>
<tr><th colspan="2">Assay compared to initial</th></tr>
<tr><th>Ivermectin</th><th>Levamisole $PO_4$</th></tr>
<tr><td>1 M</td><td>103</td><td>103</td></tr>
<tr><td>2 M</td><td>103</td><td>99</td></tr>
<tr><td>3 M</td><td>100</td><td>95</td></tr>
<tr><td>6 M</td><td>101</td><td>94</td></tr>
<tr><td>9 M</td><td>93</td><td>90</td></tr>
</table>
</td></tr>
<tr><td>INJ003 -10</td><td>1500 mL</td><td>Ivermectin<br>Levamisole $PO_4$<br>Capmul MCM<br>Tween 80<br>BHT<br>Methylparaben<br>Propylparaben<br>Tri Sodium Citrate<br>Propyl gallate<br>Conc. HCl<br>WFI</td><td>0.57<br>20.0<br>25.0<br>15.0<br>0.03<br>0.18<br>0.20<br>1.5<br>0.02<br>0.25 mL<br>to 100</td><td>Purpose: Batch with Levamisole $PO_4$ & BHT with pH 4.0
<table>
<tr><th rowspan="3"></th><th colspan="2">2-8°C</th></tr>
<tr><th colspan="2">Assay compared to initial</th></tr>
<tr><th>Ivermectin</th><th>Levamisole $PO_4$</th></tr>
<tr><td>1 M</td><td>103</td><td>103</td></tr>
<tr><td>2 M</td><td>103</td><td>99</td></tr>
<tr><td>3 M</td><td>96</td><td>102</td></tr>
<tr><td>6 M</td><td>104</td><td>102</td></tr>
<tr><td>9 M</td><td>100</td><td>98</td></tr>
</table>
<table>
<tr><th rowspan="3"></th><th colspan="2">25°C</th></tr>
<tr><th colspan="2">Assay compared to initial</th></tr>
<tr><th>Ivermectin</th><th>Levamisole $PO_4$</th></tr>
<tr><td>1 M</td><td>103</td><td>102</td></tr>
<tr><td>2 M</td><td>103</td><td>99</td></tr>
<tr><td>3 M</td><td>99</td><td>96</td></tr>
<tr><td>6 M</td><td>99</td><td>94</td></tr>
<tr><td>9 M</td><td>92</td><td>89</td></tr>
</table>
</td></tr>
</table>

(continued)

| Batch No | Batch size | Formulation | % w/v | Purpose, method of manufacture and comments |
|---|---|---|---|---|
| INJ003-11 | 1500 mL | Ivermectin | 0.57 | Purpose: Batch with Levamisole $PO_4$ & BHT with pH 5.0 |
| | | Levamisole $PO_4$ | 20.0 | |
| | | Capmul MCM | 25.0 | |
| | | Tween 80 | 15.0 | |
| | | BHT | 0.03 | |
| | | Methylparaben | 0.18 | |
| | | Propylparaben | 0.20 | |
| | | Tri Sodium Citrate | 1.5 | |
| | | Propyl gallate | 0.02 | |
| | | Conc. HCl | 0.25 mL | |
| | | WFI | to 100 | |

| | 2-8°C | |
|---|---|---|
| | Assay compared to initial | |
| | **Ivermectin** | **Levamisole $PO_4$** |
| 1 M | 101 | 104 |
| 2 M | 102 | 102 |
| 3 M | 101 | 10 |
| 6 M | 102 | 100 |
| 9 M | 98 | 99 |

| | 25°C | |
|---|---|---|
| | Assay compared to initial | |
| | **Ivermectin** | **Levamisole $PO_4$** |
| 1 M | 102 | 102 |
| 2 M | 102 | 101 |
| 3 M | 95 | 96 |
| 6 M | 98 | 94 |
| 9 M | 97 | 86 |

*INJ0003/11 Method of Preparation*

1. Ivermectin was dissolved in CAPMUL MCM under stirring.
2. To this Tween 80 was added under stirring.
3. BHT was added to step 1 under stirring.
4. Methyl paraben and Propyl paraben dissolved in 600ml of water under heating.
5. Levamisole phosphate was added to step 3 under stirring at room temperature.
6. Propyl gallate was added to step 4 under stirring.
7. Trisodium citrate and 50% HCl solution were added to step 5 under stirring.
8. Step 2 was added to step 6 under stirring
9. Adjusted the pH to around 5.00 using 20% HCl solution, if necessary.
10. The resulting solution was made up to volume with water.

Table 11: Site reaction test for ivermectin / levamisole injection for cattle - INJ 0003/11

| | |
|---|---|
| Species: | Bovine |
| Number: | 2 animals |
| Type: | mixed |
| Age: | ~6 months |
| Sex: | either |
| Weight: | Greater than 125kg |
| Source: | Commercial farm |
| Health status: | Normal animals in good body condition. |
| Special requirements: | No anthelmintic treatment in the past 4 weeks |
| Medication history: | Herd-based treatments as part of normal animal husbandry practice. |
| Housing and management: | The study animals were maintained as a single mob and rotationally grazed on pasture with continuous access to water throughout the experimental period. |

*Results:* Two calves were treated. No sting in either animal was noted upon injection. A site reaction occurred in both animals within 1 hour of injection, lasting for the entire 28 days of the trial. The reaction was a slightly raised area at the point of injection getting as large as 150 x 170mm and 110 x 140mm. Such reactions would be unacceptable in a commercial farming situation.

<u>Example 4 - Formulations including other macrocyclic lactones</u>

Tests were conducted to determine whether the invention would be able to be used as a vehicle for delivering a wider range of macrocyclic lactone active ingredients. The test formulations were prepared using POLYSORBATE 80 as the surfactant of choice according to Table 12.

Table 12. Formulation ingredients

| Ingredient | % w/v Composition | Function |
|---|---|---|
| Macrocyclic lactone active | 0.70* | Active |
| Levamisole Phosphate | 22.3* | Active |
| BHT | 0.1 | Antioxidant |
| Dimethylacetamide | 5.0 | Solvent |
| Methyl Paraben | 0.15 | Preservative |
| Polysorbate 80 | 5.0 | Surfactant |
| Water For Injection | q.s. to 100% | Vehicle |
| *Corrected for potency | | |

The results demonstrated that abamectin, moxidectin and doramectin are suitable macrocyclic lactones for formulations according to the instant invention. When compared to the eprinomectin/levamisole formulation, the general physical parameters are very similar.

Table 13a: Formulations of various macrocyclic lactones levamisole

| | Eprinomectin/Levamisole Inj. | | | Abamectin/Levamisole Inj. (REF) | | |
|---|---|---|---|---|---|---|
| B.No. | SOW 21-08 | | | SOW 21-23 | | |
| Condition | 2-8°C | 25°C | 40°C | 2-8°C | 25°C | 40°C |
| Storage Period | 5 Days | 5 Days | 5 Days | 5 Days | 5 Days | 5 Days |
| Description | * | * | * | * | * | * |

(continued)

| | Eprinomectin/Levamisole Inj. | | | Abamectin/Levamisole Inj. (REF) | | |
|---|---|---|---|---|---|---|
| B.No. | SOW 21-08 | | | SOW 21-23 | | |
| Wt/mL | 1.0843 | 1.0843 | 1.0843 | 1.0831 | 1.0831 | 1.0831 |
| Viscosity | 3.5 cP | 3.5 cP | 3.5 cP | 3.3 cP | 3.3 cP | 3.3 cP |
| pH | 4.22 | 4.21 | 4.06 | 4.17 | 4.16 | 4.05 |

Table 13b (REF): Formulations of various macrocyclic lactones levamisole

| | Moxidectin/Levamisole Inj. | | | Doramectin/Levamisole Inj. | | |
|---|---|---|---|---|---|---|
| | Inj. | | | Inj. | | |
| B.No. | SOW 21-24 | | | SOW 21-25 | | |
| Condition | 2-8°C | 25°C | 40°C | 2-8°C | 25°C | 40°C |
| Storage Period | 5 Days | 5 Days | 5 Days | 5 Days | 5 Days | 5 Days |
| Description | * | * | * | * | * | * |
| Wt/mL | 1.0825 | 1.0825 | 1.0825 | 1.0837 | 1.0837 | 1.0837 |
| Viscosity | 4.2 cP | 4.2 cP | 4.2 cP | 3.3 cP | 3.3 cP | 3.3 cP |
| pH | 4.15 | 4.14 | 4.02 | 4.13 | 4.12 | 4.01 |
| * Clear very light yellow solution | | | | | | |

Example 5 - Other Formulations

Formulations including novel avermectins would be prepared in accordance with Table 12, and could include the compounds and amounts recited in Table 14. The formulations could also include any one of the avermectins or avermectin monosaccharides, modified in the 4' or 4" position, described in US 7,704,961, US 7,521,429, US 2006-0105970 A1, US 7,678,740, US 7,632,820, US 7,678,773, US 7,605,134, and US 6,933,260 (each to Merial).

Table 14. Amounts and identities of novel avermectin compounds

| Avermectin | % w/v Composition |
|---|---|
| 4"-diallylaminosulfonyloxy-avermectin $B_1$ | 0.5 - 0.9 |
| 4"-(2-Hydroxyethylaminosulfonyloxy)-avermectin $B_1$ | 0.5 - 0.9 |
| 4"-dipropargylaminosulfonyloxy-avermectin $B_1$ | 0.5 - 0.9 |
| 4"-bis(cyanomethyl)aminosulfonyloxy-avermectin $B_1$ | 0.5 - 0.9 |
| 4"-Dicyanomethylaminosulfonyloxy-avermectin $B_1$ | 0.5 - 0.9 |
| 4"-pyrrolidinosulfonyloxy-avermectin $B_1$ | 0.5 - 0.9 |
| 4"-benzoylaminosulfonyloxy-avermectin $B_1$ | 0.5 - 0.9 |
| 4"-epi-sulfamoyloxy-avermectin $B_1$ | 0.5 - 0.9 |
| 4"-methylaminosulfonyloxy-avermectin $B_1$ | 0.5 - 0.9 |
| 4"-ethylaminosulfonyloxy-avermectin $B_1$ | 0.5 - 0.9 |
| 4"-sulfamoyloxy-avermectin $B_1$ | 0.5 - 0.9 |
| 4"-allylaminosulfonyloxy-avermectin $B_1$ | 0.5 - 0.9 |
| Any dimeric avermectin<br>Any hetero-dimeric avermectin/milbemycin | 0.5 - 0.9 |

(continued)

| Avermectin | % w/v Composition |
|---|---|
| Any 4" substituted avermectin | 0.5 - 0.9 |
| Any 4'-4" dually substituted avermectin | 0.5 - 0.9 |
| Any avermectin monosaccharide | 0.5 - 0.9 |

Example 6 - Determination of the chemical residues and depletion profiles for eprinomectin and levamisole following subcutaneous injection of cattle.

These studies were conducted to determine the residue levels and depletion profile of eprinomectin and levamisole in the tissue of cattle, following the subcutaneous injection of an injectable formulation according to the instant invention, containing 7 g/L eprinomectin and 223 g/L levamisole phosphate. Thirty Short Horn cross cattle were sourced from a commercial farm. On the day of treatment (Day 0) all cattle were weighed, and then, based on live weight, 24 animals were allocated to treatment and tissue collection groups. The treatment groups and sampling times are presented in Table 15.

Table 15. Treatment groups, active ingredients and dose rates

| **Treatment group** | **Active ingredient concentration** | **Dose rate** | **Number of cattle treated (tissue collection times)** |
|---|---|---|---|
| 1. Untreated | - | - | 2 cattle (2 on Day 21) |
| 2. Test Item | 7 g/L eprinomectin + 223 g/L levamisole phosphate | 1.25 mL/35 kg | 20 cattle (5 on each of Days 21, 28, 35 and 42) |
| 3. Test Item contingency | 7 g/L eprinomectin + 223 g/L levamisole phosphate | 1.25 mL/35 kg | 2 cattle |

*Residues*. The untreated cattle were removed from the site of treatment before application of the test item. A single injection was made on Day 0 using pre-calibrated plastic graduated disposable syringes and 18 G x 1" hypodermic needles. The calculated dose was administered subcutaneously into the neck (20 cm above and on an angle of approximately 45 degrees from the shoulder). The following biological samples were collected on Days 21, 28, 35 and 42, and were submitted to the analytical laboratory for residue analysis: injection site, muscle (*latissimus dorsi*), kidney, liver and peri-renal fat. Both primary and reserve samples were stored frozen until shipment of the "primary samples" to the lab. "Reserve" samples remained in frozen storage. No detectable concentrations of eprinomectin nor levamisole were found in any tissues from untreated cattle indicating no contamination at the field or analytical phases. Statistical analyses and withholding periods were calculated. No adverse effects were noted in any of the treated cattle for the duration of the study.

Table 16. Study design

| **Parameter** | **Description** |
|---|---|
| Number | 24 |
| Species | Bovine |
| Breed | Short Horn cross (Hereford x Friesian dams, Short Horn sires) |
| Sex | Females and castrated males |
| Age | Approximately 8 Months |
| Body weight Range | 200-246 kg |
| Condition | Excellent condition, healthy |

The field study was conducted according to the details outlined in Table 16. In this GLP tissue residue study, twenty (20) treated cattle and two (2) untreated control cattle were sacrificed for tissue collection following the subcutaneous injection of a developmental parasiticide. Two (2) further "contingency" animals were treated and managed with the treated mob. All animals were chosen from a mob of thirty (30) cattle. Upon arrival at the research facility, all cattle were already individually identified with an ear tag with a unique number in their left ear so it was unnecessary to remove that tag and/or replace it with another. On the day of treatment (Day 0) all cattle were weighed and ranked from highest to lowest based on live weight. In order to keep the live weight range as narrow as possible, the heaviest four and lightest two animals from the 30 potential study animals were removed from the study. Labelled discs (20 x treated, 2 x contingency and 2 x untreated) were drawn randomly from a box and assigned to each animal as per ranking, in order of drawing. This meant that the cattle were randomly allocated to treatment groups (treated or untreated). Slaughter days were randomly allocated to treated cattle in blocks of four. Average live weights for the treatment group by collection day are shown in Table 17.

Table 17. Average live weight of each tissue collection day for treated and untreated cattle

| Treatment Group | | Collection day | | | |
|---|---|---|---|---|---|
| | 0 | 21 | 28 | 35 | 42 |
| Untreated | | 223 | | | |
| Treated | | 231 | 228 | 227 | 227 |
| Contingency | 215 | | | | |

Cattle that were treated received a white uniquely numbered tag in their right ear while the untreated control cattle received a red uniquely numbered tag in their right ear. Contingency animals received a blue numbered tag in their right ear. Treatment details are presented in Table 18.

Table 18. Active ingredient, dose rates and cattle numbers in treatment groups in accordance with ACVAM guidelines for maximum allowable residues in animal meat, edible offal and fat tissue

| Treatment group | Active ingredient concentration | Dose rate | | Number of cattle |
|---|---|---|---|---|
| 1. Untreated | - | | - | 2 |
| 2. Test Item | 7 g/L eprinomectin and 223 g/L levamisole phosphate | 1.25mL/35 kg | | 20 |
| 3. Contingency | 7 g/L eprinomectin and | 1.25mL/35 kg | | 2 |
| | 223 g/L levamisole phosphate | | | |

Table 19. Presents the maximum residue limits for eprinomectin (mg/kg), and levamisole (mg/kg) limit (PQL) for eprinomectin (mg/kg) and levamisole (mg/kg) in animal meat, edible offal and fat tissue

| Eprinomectin (mg/kg) | | Levamisole (mg/kg) |
|---|---|---|
| Fat | 0.25 | 0.01 |
| Liver | 1.5 | 0.1 |
| Kidney | 0.3 | 0.01 |
| Muscle | 0.05 | 0.01 |

Table 20. Limit of quantitation (LOQ), limit of detection (LOD) and practical quantitation limit (PQL) for eprinomectin (mg/kg) and levamisole (mg/kg) in animal meat, edible offal and fat tissue

| Eprinomectin (mg/kg) | | | | Levamisole (mg/kg) | | |
|---|---|---|---|---|---|---|
| LOD LOQ PQL | | | | LOD LOQ PQL | | |
| Fat tissue | 0.001 | 0.005 | 0.01 | 0.001 | 0.005 | 0.01 |
| Edible offal & meat | 0.001 | 0.005 | 0.01 | 0.002 | 0.006 | 0.01 |

Table 21. Eprinomectin B1a and levamisole concentrations in **liver** tissue

| Ear Tag | Interval | Product | Eprinomectin B1a (mg/kg) | | Levamisole (mg/kg) | |
|---|---|---|---|---|---|---|
| | | | (Corrected) | (Uncorrected) | (Corrected) | (Uncorrected) |
| 1 | 21 | UTC | < LOD | < LOD | < LOD | < LOD |
| 2 | 21 | UTC | < LOD | < LOD | < LOD | < LOD |
| 3 | 21 | Eprinomectin/Levamisole | 0.21 | 0.22 | 0.011 | 0.01 |
| 4 | 21 | Eprinomectin/Levamisole | 0.15 | 0.16 | < LOQ | < LOQ |
| 5 | 21 | Eprinomectin/Levamisole | 0.024 | 0.024 | < LOQ | < LOQ |
| 6 | 21 | Eprinomectin/Levamisole | 0.023 | 0.023 | < LOQ | < LOQ |
| 7 | 21 | Eprinomectin/Levamisole | 0.022 | 0.023 | < LOQ | < LOQ |
| 8 | 28 | Eprinomectin/Levamisole | 0.013, 0.013 | 0.013, 0.014 | < LOQ, < LOQ | < LOQ, < LOQ |
| 9 | 28 | Eprinomectin/Levamisole | 0.04 | 0.04 | < LOQ | < LOQ |
| 10 | 28 | Eprinomectin/Levamisole | 0.048 | 0.049 | < LOQ | < LOQ |
| 11 | 28 | Eprinomectin/Levamisole | < LOD | < LOD | < LOD | < LOD |
| 12 | 28 | Eprinomectin/Levamisole | 0.099 | 0.009 | < LOD | < LOQ |
| 13 | 35 | Eprinomectin/Levamisole | < LOQ | < LOQ | < LOQ | < LOQ |
| 14 | 35 | Eprinomectin/Levamisole | 0.019 | 0.019 | < LOD | < LOD |
| 15 | 35 | Eprinomectin/Levamisole | 0.012 | 0.013 | < LOD | < LOD |
| 16 | 35 | Eprinomectin/Levamisole | < LOD | < LOD | < LOD | < LOD |
| 17 | 35 | Eprinomectin/Levamisole | 0.005 | 0.005 | < LOD | < LOD |
| 18 | 42 | Eprinomectin/Levamisole | < LOD, < LOD | < LOD, < LOD | < LOQ, < LOQ | < LOQ, < LOQ |
| 19 | 42 | Eprinomectin/Levamisole | < LOD | < LOD | < LOQ | < LOQ |
| 20 | 42 | Eprinomectin/Levamisole | < LOD | < LOD | < LOQ | < LCQ |
| 21 | 42 | Eprinomectin/Levamisole | < LOD | < LOD | < LOQ | < LOQ |
| 22 | 42 | Eprinomectin/Levamisole | < LOD | < LOD | < LOD | < LOD |

Table 22. Eprinomectin B1a and levamisole concentrations in **kidney** tissue

| Ear Tag | Interval | Product | Eprinomectin B1a (mg/kg) | | Levamisole (mg/kg) | |
|---|---|---|---|---|---|---|
| | | | (Corrected) | (Uncorrected) | (Corrected) | (Uncorrected) |
| 1 | 21 | UTC | < LOD | < LOD | < LOD | < LOD |
| 2 | 21 | UTC | < LOD, < LOD | < LOD, < LOD | < LOD, < LOD | < LOD, LOD |
| 3 | 21 | Eprinomectin/Levamisole | 0.044 | 0.045 | < LOD | < LOD |
| 4 | 21 | Eprinomectin/Levamisole | 0.02 | 0.02 | < LOO | < LOD |
| 5 | 21 | Eprinomectin/Levamisole | 0.006 | 0.006 | < LOD | < LOD |
| 6 | 21 | Eprinomectin/Levamisole | < LOQ | < LOQ | < LOD | < LOD |
| 7 | 21 | Eprinomectin/Levamisole | < LOQ, < LOQ | < LOQ, LOQ | < LOD, < LOD | < LOD, < LOD |
| 8 | 28 | Eprinomectin/Levamisole | < LOQ | < LOQ | < LOD | < LOD |
| 9 | 28 | Eprinomectin/Levamisole | 0.005 | 0.006 | < LOD | < LOD |
| 10 | 28 | Eprinomectin/Levamisole | 0.006 | 0.007 | < LOD | < LOD |
| 11 | 28 | Eprinomectin/Levamisole | < LOD | < LOD | < LOD | < LOD |
| 12 | 28 | Eprinomectin/Levamisole | < LOD | < LOD | < LOD | < LOD |
| 13 | 35 | Eprinomectin/Levamisole | < LOD | < LOD | < LOD | < LOD |
| 14 | 35 | Eprinomectin/Levamisole | < LOQ | < LOQ | < LOD | < LOD |
| 15 | 35 | Eprinomectin/Levamisole | < LOQ | < LOQ | < LOD | < LOD |
| 16 | 35 | Eprinomectin/Levamisole | < LOD | < LOD | < LOD | < LOD |
| 17 | 35 | Eprinomectin/Levamisole | < LOD, < LOD | < LOS, < LOD | < LOD, < LOD | < LOD, < LOD |
| 18 | 42 | Eprinomectin/Levamisole | < LOD | < LOD | < LOD | < LOD |
| 19 | 42 | Eprinomectin/Levamisole | < LOD | < LOD | < LOD | < LOD |

(continued)

| Ear Tag | Interval | Product | Eprinomectin B1a (mg/kg) | | Levamisole (mg/kg) | |
|---|---|---|---|---|---|---|
| | | | (Corrected) | (Uncorrected) | (Corrected) | (Uncorrected) |
| 20 | 42 | Eprinomectin/Levamisole | < LOD | < LOD | < LOD | < LOD |
| 21 | 42 | Eprinomectin/Levamisole | < LOD | < LOD | < LOD | < LOD |
| 22 | 42 | Eprinomectin/Levamisole | < LOD | < LOD | < LOD | < LOD |

Table 23. Eprinomectin B1a and levamisole concentrations **in injection** site tissue

| Ear Tag | Interval | Product | Eprinomectin B1a (mg/kg) | | Levamisole (mg/kg) | |
|---|---|---|---|---|---|---|
| | | | (Corrected) | (Uncorrected) | (Corrected) | (Uncorrected) |
| 1 | 21 | UTC | < LOD | < LOD | < LOD | < LOD |
| 2 | 21 | UTC | < LOD | < LOD | < LOD | < LOD |
| 3 | 21 | Eprinomectin/Levamisole | 0.12 | 0.12 | < LOQ | < LOQ |
| 4 | 21 | Eprinomectin/Levamisole | 0.015 | 0.015 | < LOD | < LOD |
| 5 | 21 | Eprinomectin/Levamisole | < LOD | < LOD | < LOD | < LOD |
| 6 | 21 | Eprinomectin/Levamisole | < LOQ | < LCQ | < LOD | < LOD |
| 7 | 21 | Eprinomectin/Levamisole | < LOD | < LOD | < LOD | < LOD |
| 8 | 28 | Eprinomectin/Levamisole | < LOD, < LOQ | < LOD, < LOQ | < LOD, < LOD | < LOD, < LOD |
| 9 | 28 | Eprinomectin/Levamisole | 0.11 | 0.11 | < LOD | < LOD |
| 10 | 28 | Eprinomectin/Levamisole | < LOQ | < LOQ | < LOD | < LOD |
| 11 | 28 | Eprinomectin/Levamisole | < LOD | < LOD | < LOD | < LOD |
| 12 | 28 | Eprinomectin/Levamisole | < LOQ | < LOQ | < LOD | < LOD |
| 13 | 35 | Eprinomectin/Levamisole | < LOD | < LOD | < LOD | < LOD |
| 14 | 35 | Eprinomectin/Levamisole | 0.005 | 0.006 | < LOD | < LOD |
| 15 | 35 | Eprinomectin/Levamisole | < LOQ | < LOQ | < LOD | < LOD |
| 16 | 35 | Eprinomectin/Levamisole | < LOQ | < LOQ | < LOD | < LOD |
| 17 | 35 | Eprinomectin/Levamisole | < LOD | < LOD | < LOD | < LOD |
| 18 | 42 | Eprinomectin/Levamisole | < LOD, LOD | < LOD, < LOD | < LOD, < LOD | < LOD, < LOD |
| 19 | 42 | Eprinomectin/Levamisole | < LOD | < LOD | < LOD | < LOD |
| 20 | 42 | Eprinomectin/Levamisole | < LOD | < LOD | < LOD | < LOD |
| 21 | 42 | Eprinomectin/Levamisole | < LOD | < LOD | < LOD | < LOD |
| 22 | 42 | Eprinomectin/Levamisole | < LOD | < LOD | < LOD | < LOD |

Table 24. Eprinomectin B1a and levamisole concentrations in peri-renal tissue of treated and untreated cattle

| Ear Tag | Interval | Product | Eprinomectin B1a (mg/kg) | | Levamisole (mg/kg) | |
|---|---|---|---|---|---|---|
| | | | (Corrected) | (Uncorrected) | (Corrected) | (Uncorrected) |
| 1 | 21 | UTC | < LOD | < LOD | < LOD | < LOD |
| 2 | 21 | UTC | < LOD | < LOD | < LOD | < LOD |
| 3 | 21 | Eprinomectin/Levamisole | < LOQ | < LOQ | < LOD | < LOD |
| 4 | 21 | Eprinomectin/Levamisole | < LOD | < LOD | < LOD | < LOD |
| 5 | 21 | Eprinomectin/Levamisole | < LOD | < LOD | < LOD | < LOD |
| 6 | 21 | Eprinomectin/Levamisole | < LOD | < LOD | < LOD | < LOD |
| 7 | 21 | Eprinomectin/Levamisole | < LOD | < LOD | < LOD | < LOD |
| 8 | 28 | Eprinomectin/Levamisole | < LOD, < LOD | < LOD, < LOD | < LOD, < LOD | < LOD, < LOD |
| 9 | 28 | Eprinomectin/Levamisole | < LOD | < LOD | < LOD | < LOD |
| 10 | 28 | Eprinomectin/Levamisole | < LOD | < LOD | < LOD | < LOD |
| 11 | 28 | Eprinomectin/Levamisole | < LOD | < LOD | < LOD | < LOD |

(continued)

| | | | Eprinomectin B1a (mg/kg) | | Levamisole (mg/kg) | |
|---|---|---|---|---|---|---|
| Ear Tag | Interval | Product | (Corrected) | (Uncorrected) | (Corrected) | (Uncorrected) |
| 12 | 28 | Eprinomectin/Levamisole | < LOD | < LOD | < LOD | < LOD |
| 13 | 35 | Eprinomectin/Levamisole | < LOD | < LOD | < LOD | < LOD |
| 14 | 35 | Eprinomectin/Levamisole | < LOD | < LOD | < LOD | < LOD |
| 15 | 35 | Eprinomectin/Levamisole | < LOD | < LOD | < LOD | < LOD |
| 16 | 35 | Eprinomectin/Levamisole | < LOD | < LOD | < LOD | < LOD |
| 17 | 35 | Eprinomectin/Levamisole | < LOD | < LOD | < LOD | < LOD |
| 18 | 42 | Eprinomectin/Levamisole | < LOD, < LOD | < LOD, < LOD | < LOD, < LOD | < LOD, < LOD |
| 19 | 42 | Eprinomectin/Levamisole | < LOD | < LOD | < LOD | < LOD |
| 20 | 42 | Eprinomectin/Levamisole | < LOD | < LOD | < LOD | < LOD |
| 21 | 42 | Eprinomectin/Levamisole | < LOD | < LOD | < LOD | < LOD |
| 22 | 42 | Eprinomectin/Levamisole | < LOD | < LOD | < LOD | < LCD |

Example 7 - Efficacy of a combination eprinomectin and levamisole injection against sucking lice *(Linognathus vituli)* in cattle.

Four species of louse have been recorded on cattle in New Zealand. Of these, the biting louse *Bovicola bovis* and one of the three species of sucking louse, *Linognathis vituli* are common and dual infections with *L. vituli* and *B. bovis* occur. The remaining two species of sucking louse, *Haematopinus eurysternus* and *Solenopotes capillatus* are uncommon (Chalmers and Charleston, 1980). The 56 day post-treatment assessment period covered at least two complete lifecycles of the louse.

This study was a randomized efficacy study in naturally infected animals with a negative control group. Prior to the start of the study, a mob of suitably infected animals were identified on a commercial grazing property located in the Waikato region of New Zealand. On Day -13 sixteen animals from the larger mob were purchased and relocated to the study site and identified using uniquely numbered ear tags. Samples of biting and sucking lice were collected from seven study animals and identified by the Study Investigator. On Day 0 all sixteen study animals were lice counted and allocated to two groups (n=8) based on individual sucking lice counts. All study animals were weighed and treatments were administered subcutaneously. Group 1 was treated with the instant eprinomectin (7g/L) / levamisole phosphate (223g/L) formulation at 1mL/35kg, and Group 2 remained untreated as the negative control group for the study. Individual lice counts were performed on each study animal on Days 7, 13 and 48. Group 1 was counted prior to Group 2, to ensure no transmission of lice between untreated animals and animals in the treated group. Injection sites were inspected on Days 7, 13 and 48 post-treatment for lesions or abnormalities.

Table 25. Treatment regime

| Group | No. Animals | Treatment | Dose | Route |
|---|---|---|---|---|
| **1** | 10 | Eprinomectin/levarnisole injection | 1mL/35kg | Subcutaneous |
| **2** | 10 | Dectamax® injectable endectocide | 1mL/50kg | Subcutaneous |
| **3** | 10 | Negative control | NA | NA |

*Statistical methods*. The efficacy of the Investigational Veterinary Product (IVP, i.e. the eprinomectin / levamisole injectable formulation recited above) against both sucking and biting lice at each time point was calculated as the percent reduction in arithmetic and geometric mean lice count on the treated group compared to the untreated control group using Equation 1 and Equation 2, as recommended in WAAVP guidelines.

Equation 1: Abbott's formula

$$\text{Efficacy } \% = \left(1 - \frac{\text{n in T after treatment}}{\text{n in Co after treatment}}\right) * 100$$

Equation 2: Henderson-Tilton's formula

$$\text{Efficacy } \% = \left(1 - \frac{\text{n in Co before treatment} * \text{n in T after treatment}}{\text{n in Co after treatment} * \text{n in T before treatment}}\right) * 100$$

Where Efficacy % = efficacy, n = mean number of lice of the relevant species, T = Treatment group and Co = Control group. All statistical analyses were performed using Minitab® for Windows® Release 16.1.0 (Minitab, Inc.). Where data were considered to be normally distributed, as determined by Levene's test of equal variance, means were compared using a Two Sample T -Test. Data that were not normally distributed were compared using an equivalent non-parametric method. Results were considered significant at P<0.05.

Tables 26 and 27 show arithmetic and geometric mean sucking and biting lice counts recorded for Groups 1 and 2 throughout the study. Both biting and sucking lice were present on all animals prior to treatment and at the termination of the study on Day 48. Both tables incorporate the results of the statistical evaluation.

Table 26. Arithmetic and geometric mean suckling lice counts

| Arithmetic mean sucking lice | | | | |
|---|---|---|---|---|
| **Group** | **Day 0** | **Day 7** | **Day 13** | **Day 48** |
| 1 | $36.3^{a}$ | $5.1^{a}$ | $13.6^{a}$ | $12.3^{a}$ |
| 2 | $26.8^{a}$ | $32.3^{b}$ | $32.1^{a}$ | $13.3^{a}$ |
| Geometric mean sucking lice | | | | |
| **Group** | **Day 0** | **Day 7** | **Day 13** | **Day 48** |
| 1 | $27.6^{a}$ | $3.8^{a}$ | $5.8^{a}$ | $10.8^{a}$ |
| 2 | $24.6^{a}$ | $30.6^{b}$ | $31.0^{b}$ | $16.2^{a}$ |
| $^{ab}$ means within the same column with the same superscript are not significantly different at p<0.05 | | | | |

Table 27. Arithmetic and geometric mean biting lice counts

| Arithmetic mean biting Lice | | | | |
|---|---|---|---|---|
| **Group** | **Day 0** | **Day 7** | **Day 13** | **Day 48** |
| 1 | $673^{a}$ | $12.4^{a}$ | $21.8^{a}$ | $17.1^{a}$ |
| 2 | $64.9^{a}$ | $46.6^{b}$ | $43.9^{b}$ | $26.0^{b}$ |
| Geometric mean biting Lice | | | | |
| **Group** | **Day 0** | **Day 7** | **Day 13** | **Day 48** |
| 1 | $60.5^{a}$ | $10.5^{a}$ | $20.3^{a}$ | $15.7^{a}$ |
| 2 | $58.2^{a}$ | $45.9^{b}$ | $41.2^{b}$ | $25.0^{b}$ |
| $^{ab}$ means within the same column with the same superscript are not significantly different at p<0.05 | | | | |

The efficacy of the IVP against sucking and biting lice are show in Tables 28 and 29. Each table presents calculations based on Equation 1 and Equation 2. The efficacy of the IVP against sucking lice peaked at 84% and 88%, based on arithmetic mean counts on Day 7 using Equation 1 and Equation 2, respectively. Efficacy had declined over the subsequent two counts to 36% and 53% by Day 48, based on Equation 1 and Equation 2, respectively. The efficacy of the IVP against sucking lice based on geometric mean counts peaked at 88% and 89% on Day 7, but declined to 33% and 41 % by Day 48, using the same two equations.

Table 28. Efficacy against sucking lice based on arithmetic and geometric mean lice counts calculated using Equation 1 and Equation 2.

| Arithmetic Efficacy Abbott's Formula sucking lice | | | |
|---|---|---|---|
| **Group** | **Day 7** | **Day 13** | **Day 48** |
| 2 | 0.84 | 0.58 | 0.36 |
| Arithmetic Efficacy Henderson-Tilton's Formula sucking lice | | | |
| **Group** | **Day 7** | **Day 13** | **Day 48** |
| 2 | 0.88 | 0.69 | 0.53 |
| Geometric Efficacy Abbott's Formula sucking lice | | | |
| **Group** | **Day 7** | **Day 13** | **Day 48** |
| 2 | 0.88 | 0.81 | 0.33 |
| Geometric Efficacy Henderson-Tilton's Formula sucking lice | | | |
| **Group** | **Day 7** | **Day 13** | **Day 48** |
| 2 | 0.89 | 0.83 | 0.41 |

Efficacy based on arithmetic mean lice counts against biting lice peaked at a lower level with the IVP achieving a maximum reduction of 73% and 74% in lice numbers, using Equation 1 and Equation 2, respectively and declined to 34% (Equation 1) and 36% (Equation 2) by Day 48. When efficacy was calculated using the geometric mean lice counts, maximum reductions of77% and 78% were achieved on Day 7 and had declined to 37% and 40%, based on Equation 1 and Equation 2.

Table 29. Efficacy against biting lice based on arithmetic and geometric mean lice counts calculated using Equation 1 and 2

| Arithmetic Efficacy Abbott's Formula biting lice | | | |
|---|---|---|---|
| **Group** | **Day 7** | **Day 13** | **Day 48** |
| 2 | 0.73 | 0.50 | 0.34 |
| Arithmetic Efficacy Henderson-Tilton's Formula biting lice | | | |
| **Group** | **Day 7** | **Day 13** | **Day 48** |
| 2 | 0.74 | 0.52 | 0.36 |
| Geometric Efficacy Abbott's Formula biting lice | | | |
| **Group** | **Day 7** | **Day 13** | **Day 48** |
| 2 | 0.77 | 0.49 | 0.37 |
| Geometric Efficacy Henderson-Tilton's Formula biting lice | | | |
| **Group** | **Day 7** | **Day 13** | **Day 48** |
| 2 | 0.78 | 0.51 | 0.40 |

As expected, the IVP was more effective against sucking lice than biting lice, given that its route of administration was by subcutaneous injection. The highest level of efficacy against sucking lice (89%) was recorded on Day 7 compared to a 78% reduction in biting lice numbers at the same time point. At 13 days post treatment, the highest level of efficacy against sucking lice was 83% compared to 52% against biting lice.

Example 8 - Safety of eprinomectin/levamisole formulations

Thirty male Hereford x Friesian cattle, each weighing between 105-123kgs, were treated according to preceding examples. The animals were healthy and had not been treated with a macrocyclic lactone or levamisole within 3 months prior to Study Day 0. One group received the 1x concentration of eprinomectin and levamisole phosphate (see Table 18) administered by subcutaneous injection, while the other group received 3x this concentration. Animals were monitored for pain and swelling, and the results indicated the eprinomectin / levamisole injection is safe to administer the recommended dose, or at least up to 3x the recommended dose (for example, in the event of an accidental overdose).

Example 9. Fecal egg count reduction efficacy of a subcutaneous combination eprinomectin and levamisole injection against a natural infection of gastrointestinal nematodes in cattle.

This study was a randomized fecal egg count reduction field efficacy study in animals carrying a natural infection, and included positive and negative control groups for comparison. Seven days prior to commencement of the study, ten individual unidentified fecal samples were collected from the paddock and individual fecal egg counts were conducted to confirm infection. Four days prior to commencement of the study, individual fecal samples were collected per rectum from 60 animals otherwise meeting the inclusion criteria. Fecal egg counts (FECs) and a pooled larval culture were performed. Forty-five animals with positive FECs were selected for the study and randomly allocated to three groups each of 15 animals based on FEC. They remained at pasture in a single mob on the source farm for the duration of the study.

On Day 0, the animals were weighed and a fecal sample was collected from each animal per rectum for individual FECs and a pooled larval culture. Group 1 was treated with the IVP, Group 2 was treated with Control Product (CP) and Group 3 remained untreated as the negative control. Treated animals were observed at 30 minutes and 2 hours after treatment. On Days 14, 16 and 28 post-treatment, fecal samples were collected per rectum for individual FECs and pooled group larval cultures. No sample was collected from Animal #60 (Group 3) on Day 14. Injection sites of animals in Group 1 were examined on Days 14 and 28. Further samples were due to be collected on days 42 and 56 but the study was concluded after day 28 due to evidence of nematode resistance to macrocyclic lactone anthelmintics. After the conclusion of the study, the study animals were treated with an oral abamectin, oxfendazole and levamisole anthelmintic (MATRIX C HI-MIN; Merial Ancare).

*Randomization Procedure*. FECs were determined for 60 animals on Day -4. Fourteen animals were excluded for negative FECs and one animal with a FECs of 1050 eggs per gram was excluded. The remaining 45 animals with positive FECs (range 50-550 eggs per gram) were selected for the study. They were ranked in ascending order of FECs and blocked into blocks of 3 animals. A random number was allocated to each animal using Microsoft Excel random number generation. The animals were ranked in ascending order of random number within each block. The animal with the lowest random number in each block was allocated to Group 1, the next to Group 2 and the highest to Group 3. Each of the three groups comprised 15 animals and had a similar average FECs.

*Blinding/masking procedure*. Laboratory staff performing FECs and group coprocultures were advised which group each animal was in after Day 0, but were not aware of the treatment allocated to each group. Forty-five Friesian/Jersey cross cattle, aged 10 months, female, and weighing on average 158.6 kg (range 106.5-208.5 kg) on Day 0, were used for the study. All animals were FEC positive, which was determined by the presence in the animals of a mean egg per gram fecal (epg) of 206, with the range being 50-550 epg on Day -4. No anthelmintic had been administered to the cattle within 30 days prior to Study Day 0. Study animals were transferred from the home dairy farm to the study site (a grazing block) approximately 6 weeks prior to Day 0. On Day 0 they were harboring an existing gastrointestinal nematode infection derived from the study site farm and/or the home dairy farm. The IVP (Eprinomectin 7g/L and levamisole phosphate 223 g/L) was administered at 1 mL/35 kg via subcutaneous route. The Control Product (CP) was DECTOMAX® (Pfizer Animal Health), which consists of 10mg/mL doramectin was administered to the animals via subcutaneous route at a dose of 1mL/50kg. Meat and milk withholding were each 35 days.

*Method of administration/application.* The IVP and CP were administered 1 time at Day 0 by subcutaneous injection in the anterior part of the neck. Individual doses were drawn up into a graduated 10mL syringe immediately prior to treatment and were checked by a second person before administration. Doses of IVP and CP were calculated on the basis of the heaviest bodyweight in the relevant group as determined on Day o. Calculated doses were rounded up to the nearest minor increment of the syringe. The IVP was administered at 1mL / 35kg. The heaviest animal in Group 1 weighed 208.5 kg and the dose of IVP administered to each animal in Group 1 was 6.0 mL. The CP was administered at 1mL/50kg. The heaviest animal in Group 2 weighed 199.0 kg and the dose of CP administered to each animal in Group 2 was 4.0 mL.

All animals were returned to the source herd at the conclusion of the study. All animals were treated with MATRIX C Hi-Min (Merial Ancare) after the conclusion of the study and relevant withholding periods applied. Efficacy of the IVP and CP was determined by fecal egg count reduction for the treated groups compared to the negative control group at each time point.

Efficacy (%E) of the IVP and CP was calculated at each time point, using the formula: %E= [C-T]/C x 100. Where: C = mean FEC of the control group; T = mean FEC of the treated group. Arithmetic and geometric mean FECs were then calculated. The significance of differences between group FECs was statistically evaluated. Where data were considered to be normally distributed as determined by Levene's test of equal variance, means were compared using One-way Analysis of Variance (ANOV A). Data that were not normally distributed were compared using an equivalent non-parametric method. Where differences were considered significant, ($p<0.05$), pair-wise comparisons were used to evaluate differences between groups.

*Results.* No injection site reactions were observed in Group 1 animals at any time point. FEC and larval culture results are shown in Tables 30-34. Table 30 shows arithmetic mean Day -4 FEC and Day 0 bodyweight. Means in the same column that share a letter (A) are not significantly different ($p<0.05$). Table 31 shows arithmetic and geometric mean FEC at Days 0 to 42. Based on arithmetic and geometric means, the FECs for Group 1 (treated with the IVP) were significantly less ($p<0.05$) than both the control group (Group 3) at every timepoint post-treatment and significantly less than Group 2 (treated with DECTOMAX) at Day 14 and 28. Based on arithmetic and geometric means, the FECs for Group 2 (treated with DECTOMAX) were not significantly different from the control group at any timepoint.

Table 30. Group details.

| Group | n | Mean (range) FEC Day -4 (epg) | Mean (range) bodyweight Day 0 (kg) |
|---|---|---|---|
| **1** | 15 | $20^{A}$(50-500) | 162 (112.5-208.5) |
| **2** | 15 | $207^{A}$(50-550) | 156 (106.5-199) |
| **3** | 15 | $207^{A}$ (50-550) | 158 (128.5-201) |

Table 31. Group FECs.

| Group | Treatment | Arithmetic mean FEC (epg) | | | |
|---|---|---|---|---|---|
| | | **D0** | **D 14** | **D 16** | **D 28** |
| **1** | **IVP** | $337^{A}$ | $37^{A}$ | $23^{A}$ | $17^{A}$ |
| **2** | **Dectomax** | $320^{A}$ | $150^{B}$ | $80^{AB}$ | $183^{a}$ |
| **3** | **Control** | $237^{A}$ | $261^{B}$ | $183^{B}$ | $180^{B}$ |
| | | **Geometric mean FEC (epg)** | | | |
| | | **D 0** | **D 14** | **D 16** | **D 28** |
| **1** | **IVP** | $219.3^{A}$ | $4.8^{B}$ | $4.0^{A}$ | $2.0^{B}$ |
| **2** | **Dectomax** | $262.6^{A}$ | $93.5^{A}$ | $27.2^{AB}$ | $44.7^{A}$ |
| **3** | **Control** | $121.8^{A}$ | $136.9^{A}$ | $87.7^{A}$ | $45.5^{A}$ |

Table 32. Percentage FEC reduction efficacy.

| Group | Treatment | % Efficacy (arithmetic means) | | | % Efficacy (geometric means) | | |
|---|---|---|---|---|---|---|---|
| | | **D 14** | **D 16** | **D 28** | **D14** | **D16** | **D28** |
| **1** | **IVP** | 859 | 87.3 | 90.7 | 96.0 | 95.4 | 95.6 |
| **2** | **Dectomax** | 42.5 | 56.4 | -1.9 | 32.0 | 69.0 | 1.8 |

Efficacy of the IVP and CP at each timepoint (based on FEC reduction compared to the untreated control group) is shown in Table 32. The percentage of each nematode genus identified in 100 larvae from group coprocultures at each timepoint is shown in Table 33.

Table 33. Larval culture results (%)

| Day | Group | Treatment | Ostertagia | Trichostrongylus | Cooperia | Number of larvae speciated |
|---|---|---|---|---|---|---|
| -4 | Pooled | NA | 50 | 7 | 43 | 100 |
| 0 | Pooled | NA | 46 | 3 | 51 | 100 |
| 14 | 1 | IVP | 100* | 0 | 0 | 1 |
| | 2 | Dectomax | 0 | 4 | 96 | 100 |
| | 3 | NA | 27 | 2 | 71 | 100 |
| 16 | 1 | IVP | 100** | 0 | 0 | 6 |
| | 2 | Dectomax | 18 | 4 | 78 | 100 |
| | 3 | NA | 30 | 1 | 69 | 100 |
| 28 | 1 | IVP | 46*** | 0 | 54*** | 35 |
| | 2 | Dectomax | 1 | 5 | 94 | 100 |
| | 3 | NA | 35 | 1 | 64 | 100 |
| *1 larva recovered ** 6 larvae recovered ***% of 35 larvae recovered | | | | | | |

The larval differential in a coproculture is usually determined from the percentage of each genus identified in the first 100 larvae counted. Small numbers of larvae were obtained from coprocultures from group 1 at days 14, 16 and 28, despite positive FECs. This suggests that egg viability or larval development may have been suppressed in this group. Individual FEC results at each timepoint are shown in Table 34.

Table 34. Individual FEC results

| Group | ID | STRONGYLE FEC(epg) | | | | |
|---|---|---|---|---|---|---|
| | | D-4 | D0 | D14 | D16 | D28 |
| 1 | 11 | 100 | 100 | 150 | 50 | 0 |
| | 15 | 150 | 100 | 0 | 0 | 0 |
| | 20 | 100 | 150 | 0 | 0 | 0 |
| | 21 | 200 | 400 | 100 | 0 | 0 |
| | 22 | 300 | 250 | 0 | 0 | 0 |
| | 35 | 400 | 1050 | 50 | 0 | 0 |
| | 36 | 50 | 200 | 0 | 0 | 100 |
| | 38 | 50 | 100 | 0 | 0 | 0 |
| | 42 | 500 | 400 | 50 | 50 | 0 |
| | 46 | 250 | 300 | 150 | 100 | 0 |
| | 47 | 150 | 100 | 0 | 50 | 0 |
| | 57 | 350 | 1500 | 0 | 50 | 50 |
| | 70 | 250 | 100 | 0 | 50 | 50 |
| | 72 | 50 | 150 | 50 | 0 | 50 |
| | 86 | 150 | 150 | 0 | 0 | 0 |

(continued)

| Group | ID | STRONGYLE FEC(epg) | | | | |
|---|---|---|---|---|---|---|
| | | D-4 | D0 | D14 | D16 | D28 |
| 2 | 3 | 200 | 550 | 100 | 200 | 150 |
| | 7 | 550 | 600 | 200 | 100 | 450 |
| | 18 | 100 | 100 | 200 | 0 | 50 |
| | 23 | 300 | 600 | 250 | 0 | 300 |
| | 26 | 100 | 200 | 50 | 50 | 50 |
| | 27 | 50 | 100 | 200 | 0 | 0 |
| | 29 | 200 | 300 | 100 | 200 | 150 |
| | 30 | 150 | 200 | 0 | 0 | 0 |
| | 55 | 50 | 200 | 150 | 50 | 350 |
| | 56 | 350 | 600 | 200 | 50 | 550 |
| | 64 | 50 | 150 | 50 | 50 | 0 |
| | 81 | 300 | 250 | 250 | 100 | 50 |
| | 91 | 350 | 600 | 50 | 100 | 400 |
| | 96 | 150 | 200 | 400 | 200 | 0 |
| | 97 | 200 | 150 | 50 | 100 | 250 |
| 3 | 13 | 50 | 50 | 50 | 0 | 0 |
| | 16 | 250 | 50 | 100 | 150 | 200 |
| | 19 | 100 | 100 | 100 | 150 | 200 |
| | 28 | 300 | 350 | 750 | 250 | 400 |
| | 33 | 550 | 1000 | 600 | 600 | 150 |
| | 40 | 200 | 300 | 250 | 250 | 150 |
| | 45 | 150 | 350 | 150 | 100 | 0 |
| | 49 | 50 | 0 | 100 | 50 | 100 |
| | 51 | 200 | 400 | 250 | 200 | 150 |
| | 53 | 100 | 100 | 150 | 0 | 0 |
| | 60 | 450 | 200 | No sample | 300 | 650 |
| | 85 | 300 | 150 | 100 | 100 | 50 |
| | 89 | 150 | 50 | 400 | 200 | 100 |
| | 94 | 50 | 100 | 0 | 100 | 0 |
| | 102 | 200 | 350 | 650 | 300 | 550 |

*Conclusions*. Based on arithmetic mean FECs, the levamisole/eprinomectin injection was over 85% effective and this effect was maintained up to 28 days after treatment. DECTOMAX (doramectin) was insufficiently effective. Based on geometric mean FECs, the levamisole/eprinomectin injection was over 95% effective and this effect was maintained up to 28 days after treatment. DECTOMAX was insufficiently effective. Efficacies were evaluated in animals carrying a mixed infection of *Ostertagia, Trichostrongylus and Cooperia* spp. There was strong evidence of anthelmintic resistance, and in particular, resistance by *Cooperia* spp. to doramectin.

Example 10 - Efficacy of a combination injectable anthelmintic containing eprinomectin and levamisole against common gastrointestinal nematodes in cattle.

*Experimental design.* This study was a randomized total worm count efficacy and dose confirmation study, with a negative control group. The study determined efficacy against a natural infection of common gastrointestinal nematodes in cattle. Prior to the study, a group of 21 animals meeting the inclusion criteria and suspected of harboring a natural infection of gastrointestinal nematodes were individually identified with uniquely numbered ear tags. The animals were fecal sampled and individual fecal egg counts (FECs) were performed. A bulk fecal sample was cultured and larval differentiation performed to identify the nematode species present. The fourteen study animals with the highest FEC results were selected for the study and introduced to the when study diet while in the paddock on day -5. On Day 0, the study animals were removed from pasture to the indoor facility. The animals were fecal sampled for individual FEC and randomly allocated into two groups of seven. One animal in each group was identified as a spare. Animals were weighed and the NP treatment was administered. Animals in Group 1 were treated with the IVP. Animals in Group 2 were left untreated. Treated animals were observed for adverse reactions at approximately 30 minutes and 1, 2 and 4 hours after treatment.

On Day 11, fecal samples were collected for individual fecal egg counts. On Day 13, animals were held off food overnight but continued to have *ad libitum* access to water. On Day 14, all animals (excluding spares) were humanely euthanized. The gastrointestinal tract of each animal was collected and forwarded directly to the laboratory for processing and subsequent worm counts. The animal phase of the study concluded after the Day 14 procedures had been performed. Bodyweights were recorded immediately prior to treatment Fecal egg count and total worm count data was entered into an excel spreadsheet for statistical analysis. Individual fecal samples were collected from study animals by rectal grab prior to the study and on Day 0, and Day 11. Samples were delivered to the Laboratory on the day of collection or the day after collection. Individual fecal egg counts were performed on samples collected prior to the study and on Day ° and 11. Bulk larval culture and larval differentiation was performed on samples collected prior to the study. Gastrointestinal tracts (GITs) were collected immediately after euthanasia. The GIT was ligated at each end of the small intestine, large intestine and abomasum. The GIT was allowed to cool at ambient temperature for approximately 2 hours after collection. Each individual GIT was sealed (together with the animal's ear tag) in an individual bag identified by the animal's ear tag number. GITs were delivered to the Analytical Laboratory as soon as possible on the day of collection, accompanied by a sample submission form. Randomization / blinding was carried out as described.

All study animals had positive FECs prior to the study with a mean egg count of 121 epg. Larvae cultured from feces from all animals sampled prior to the study showed the following parasite species, 86% *Cooperia* spp., and 14% *Ostertagia (Teladorsagia)* spp. The mean body weight of the treatment group (excluding the reserve animal) on day zero of the study was 98.0kg. The mean body weight of the control group (excluding the reserve animal) on day zero of the study was 96.5kg. IVP was prepared and administered as in Example 9, and comprised eprinomectin (at 7 g/L) and levamisole phosphate (at 223 g/L). The injection site was cleaned and dried and was clipped then swabbed with 70% alcohol before treatment. A new sterile 20G 1.5 inch needle was used for each injection. Administration was performed once during the study, on Day 0, based upon bodyweight at 1 mL/35kg bodyweight. After the conclusion of the study, the spare animals were returned to the source herd. The treated spare was subjected to observation of a 91 day withholding period.

Worm counts were performed according to the *VICH Guideline on the efficacy of anthelmintics: general requirements* (1998). Counts were made on 2% aliquots of abomasa and small intestine washings that had been sieved through a 38$\mu$m sieve. Counts were also made on 10% aliquots of large intestine washings that had been sieved through a 150$\mu$m sieve. Total Worm Counts & Fecal Egg Counts: Calculation of IVP percentage efficacy (%E) was performed for each of the worm species (WS) present using the following formula: %E (Mean of (WS) in controls - mean of (WS) in treatment group) x 100 Mean of(WS) in controls. The same %E calculations were used to analyze the fecal egg count data. Statistics were calculated as described in the preceding Examples.

*Results*. Fecal egg counts: Mean fecal egg count (FEC) results are shown in Table 35. Based on FEC results the IVP was> 99.9% effective at reducing FECs at eleven days post treatment.

Table 35. Arithmetic mean FECs and % reduction in FECs of treated animals relative to controls in calves treated with a combination eprinomectin and levamisole injection

| Study Day | Control Grp FEC (epg) | Treatment Grp FEC (epg) | Efficacy |
|---|---|---|---|
| **Day 0** | 108.3 | 91.7 | / |
| **Day 11** | 250 | 0* | >99.9% |
| * = Statistically significant difference from the control group | | | |

Table 36. Arithmetic mean worm counts and % reduction in worm numbers of treated animals relative to controls in calves treated with a combination eprinomectin and levamisole injection.

| Parasite | Control | Treatment | Efficacy |
|---|---|---|---|
| ***Ostertagia* (adult)** | 1867 | 0 | >99.9% |
| ***Ostertagia* (immature)** | 50 | 0 | >99.9% |
| ***Trichostronylus axei* (adult)** | 367 | 0 | >99.9% |
| ***Haemonchus contortus* (adult)** | 42 | 0 | >99.9% |
| ***Haemonchus contortus* (immature)** | 17 | 0 | >99.9% |
| ***Cooperia* (adult)** | 18550 | 0 | >99.9% |
| ***Cooperia* (immature)** | 192 | 0 | >99.9% |
| ***Trichostrongylus* (adult)** | 33 | 0 | >99.9% |
| ***Nematodirus* (adult)** | 17 | 0 | >99.9% |
| ***Oesophagostomum* (adult)** | 12 | 0 | >99.9% |

*Total worm counts.* Arithmetic and Geometric mean total worm counts and percentage efficacy of the treatment group relative to the control group are presented in Table 36 and 37. Results demonstrate that the IVP was highly effective in the control of a mixed infection of gastrointestinal parasites in cattle. The IVP was >99.9% effective against the following parasite spp; *Ostertagia* (adult & immature), *Trichostrongylus axei* (adult), *Haemonchus contortus* (adult & immature), *Cooperia* (adult & immature), *Trichostrongylus* (adult), *Nematodirus* (adult), *Oesophagostomum* (adult).

Table 37. Geometric mean worm counts and % reduction in worm numbers of treated animals relative to controls in calves treated with a combination eprinomectin and levamisole injection.

| Parasite | Control | Treatment | Efficacy |
|---|---|---|---|
| ***Ostertagia* (adult)** | 1338 | 0* | >99.9% |
| ***Ostertagia* (immature)** | 8 | 0 | >99.9% |
| ***Trichostronylus axei* (adult)** | 237 | 0* | >99.9% |
| ***Haemonchus contortus* (adult)** | 8 | 0 | >99.9% |
| ***Haemonchus contortus* (immature)** | 3 | 0 | >99.9% |
| ***Cooperia* (adult)** | 14015 | 0* | >99.9% |
| ***Cooperia* (immature)** | 147 | 0* | >99.9% |
| ***Trichostrongylus* (adult)** | 7 | 0 | >99.9% |
| ***Nematodirus* (adult)** | 3 | 0 | >99.9% |
| ***Oesophagostomum* (adult)** | 8 | 0* | >99.9% |
| * = Statistically significant difference from the control group | | | |

*Conclusions.* This study was designed to determine the efficacy of an eprinomectin / levamisole injection against a mixed infection of common gastrointestinal parasites in cattle. Results demonstrate that the IVP was highly efficacious. Efficacies of >99.9% were recorded against the following parasite spp; *Ostertagia* (adult and immature), *Trichostrongylus axei* (adult), *Haemonchus contortus* (adult and immature), *Cooperia* (adult and immature), *Trichostrongylus* (adult), *Nematodirus* (adult), *Oesophagostomum* (adult).

Example 11. Efficacy of investigational eprinomectin / levamisole formulation versus commercial product

This study was performed using the methods described in the preceding Examples, except that an additional DECTOMAX treatment group was added.

*Results*. No injection site reactions were observed in Group 1 animals at either the Day 14 or the Day 28 observations. Table 38 below shows group arithmetic and geometric mean FEC at Days -8 to 50. Means in the same column that do not share a letter (A/B/C) are significantly different ($p<0.05$). Based on both arithmetic and geometric means, the FECs for Group 1 (treated with the IVP) and Group 2 (treated with DECTOMAX) were significantly less ($p<0.05$) than the control group (Group 3) at each of the post-treatment timepoints up to and including Day 43. The FECs for Group 1 (treated with the IVP) were also significantly less ($p<0.05$) than the control group (Group 3) at day 50.

Table 38. Group mean FECs (epg)

| Group | Treatment | Arithmetic mean FEC | | | | | |
|---|---|---|---|---|---|---|---|
| | | **D-8** | **D 0** | **D 14** | **D 28** | **D 43** | **D50** |
| **1** | **IVP** | $146.7^{A}$ | $96.4^{A}$ | $6.7^{A}$ | $13.3^{A}$ | $13.3^{A}$ | $23.3^{A}$ |
| **2** | **Dectomax** | $140.0^{A}$ | $83.3^{A}$ | $43.3^{A}$ | $46.7^{A}$ | $46.7^{A}$ | $43.3^{AB}$ |
| **3** | **Control** | $133.3^{A}$ | $121.4^{A}$ | $166.7^{B}$ | $133.3^{B}$ | $156.7^{B}$ | $100.0^{B}$ |
| | | **Geometric mean FEC** | | | | | |
| | | **D-8** | **D 0** | **D 14** | **D 28** | **D 43** | **D50** |
| **1** | **IVP** | $134.1^{A}$ | $52.2^{A}$ | $0.4^{B}$ | $0.8^{C}$ | $1.3^{B}$ | $4.0^{B}$ |
| **2** | **Dectomax** | **$129.1^{A}$** | $30.1^{A}$ | $4.0^{B}$ | $12.0^{A}$ | $7.5^{B}$ | $9.0^{AB}$ |
| **3** | **Control** | $131.4^{A}$ | $65.0^{A}$ | $59.5^{A}$ | $87.8^{A}$ | $109.9^{A}$ | $52.4^{A}$ |

Efficacy of the IVP and CP at each timepoint (based on FEC reduction compared to the untreated control group) is shown in Table 39.

Table 39. Percentage FEC reduction efficacy

| Group | Treatment | % Efficacy (arithmetic means) | | | | % Efficacy (geometric means) | | | |
|---|---|---|---|---|---|---|---|---|---|
| | | **D14** | **D 28** | **D 43** | **D 50** | **D14** | **D28** | **D 43** | **D 50** |
| **1** | **IVP** | 96.0 | 90.0 | 91.5 | 76.7 | 99.0 | 99.1 | 98.8 | 92.3 |
| **2** | **Dectomax** | 74.0 | 65.0 | 70.2 | 56.7 | 93.0 | 86.3 | 93.2 | 82.8 |

The percentage of each nematode genus identified in 100 larvae from group coprocultures at each timepoint is shown in Table 40. Only 18 larvae were recovered and identified from the coproculture for Group 1 on Day 14. This suggests that egg viability or larval development may have been suppressed in this Group.

Table 40. Larval culture results (%).

| Day | Group | Treatment | Ostertagia | Trichostrongylus | Cooperia | Haemonchus |
|---|---|---|---|---|---|---|
| 0 | Pooled | NA | 14 | 1 | 85 | 0 |
| 14 | 1 | IVP | 100* | 0 | 0 | 0 |
| | 2 | Dectomax | 0 | 0 | 100 | 0 |
| | 3 | NA | 28 | 13 | 59 | 0 |
| 28 | 1 | IVP | 86 | 0 | 14 | 0 |
| | 2 | Dectomax | 0 | 0 | 100 | 0 |
| | 3 | NA | 19 | 0 | 81 | 0 |

(continued)

| Day | Group | Treatment | Ostertagia | Trichostrongylus | Cooperia | Haemonchus |
|---|---|---|---|---|---|---|
| 43 | I | IVP | 41 | 0 | 59 | 0 |
| | 2 | Dectomax | 6 | 2 | 92 | 0 |
| | 3 | NA | 19 | 0 | 81 | 0 |
| 50 | 1 | IVP | 14 | 2 | 84 | 0 |
| | 2 | Dectomax | 22 | 0 | 78 | 0 |
| | 3 | NA | 34 | 6 | 60 | 0 |
| *18 larvae recovered. | | | | | | |

Table 41 shows group body weights at Day 0 and Day 50. Means in the same column that do not share a letter (A/B) are significantly different (p<0.05). The mean live weight gain for Group 1 (treated with the IVP) over the 50 day period of the study was significantly greater than for the untreated control group (Group 3).

Table 41. Bodyweights

| Group | Treatment | Mean (range) bodyweight Day 0 (kg) | Mean (range) bodyweight Day 50 (kg) | Mean (range) weight gain (kg) |
|---|---|---|---|---|
| **1** | **IVP** | 177.2 (138-221) | 233.5 (201-266) | $56.3^{A}$ (43-80) |
| **2** | **Dectomax** | 184.1 (128-229) | 234.5 (153-285) | $50.4^{AB}$ (25-71) |
| **3** | **Control** | 189.6 (163-234) | 235.4 (218-291) | $45.8^{B}$ (33-58) |

Table 42. Individual FEC results

| Group | ID | STRONGYLE FEC (epg) | | | | | |
|---|---|---|---|---|---|---|---|
| | | D-8 | D0 | D14 | D28 | D43 | D50 |
| **1** | **101** | 100 | 100 | 0 | 0 | 0 | 50 |
| | **103** | 150 | 200 | 0 | 50 | 50 | 0 |
| | **112** | 100 | 150 | 0 | 0 | 100 | 50 |
| | **113** | 200 | 50 | 0 | 0 | 0 | 100 |
| | **116** | 150 | 50 | 100 | 150 | 0 | 50 |
| | **118** | 100 | 100 | 0 | 0 | 0 | 50 |
| | **126** | 400 | 0 | 0 | 0 | 0 | 0 |
| | **127** | 100 | 100 | 0 | 0 | 0 | 0 |
| | **128** | 100 | 150 | 0 | 0 | 0 | 0 |
| | **138** | 150 | 50 | 0 | 0 | 0 | 0 |
| | **143** | 100 | 100 | 0 | 0 | 0 | 50 |
| | **149** | 150 | 150 | 0 | 0 | 0 | 0 |
| | **153** | 200 | NS | 0 | 0 | 50 | 0 |
| | **158** | 100 | 0 | 0 | 0 | 0 | 0 |
| | **163** | 100 | 150 | 0 | 0 | 0 | 0 |

(continued)

| Group | ID | STRONGYLE FEC (epg) | | | | | |
|---|---|---|---|---|---|---|---|
| | | D-8 | D0 | D14 | D28 | D43 | D50 |
| **2** | **102** | 150 | 100 | 0 | 50 | 50 | 0 |
| | **111** | 100 | 100 | 0 | 100 | 100 | 0 |
| | **114** | 100 | 50 | 100 | 50 | 0 | 0 |
| | **119** | 300 | 0 | 0 | 0 | 100 | 150 |
| | **121** | 100 | 100 | 100 | 100 | 0 | 100 |
| | **123** | 200 | 100 | 0 | 0 | 100 | 50 |
| | **124** | 150 | 0 | 100 | 50 | 0 | 50 |
| | **129** | 50 | 150 | 0 | 50 | 0 | 50 |
| | **136** | 100 | 0 | 0 | 0 | 0 | 0 |
| | **137** | 200 | 150 | 200 | 150 | 150 | 0 |
| | **139** | 150 | 100 | 0 | 0 | 0 | 50 |
| | **146** | 100 | 100 | 0 | 50 | 0 | 100 |
| | **150** | 100 | 0 | 150 | 100 | 0 | 100 |
| | **160** | 150 | 100 | 0 | 0 | 100 | 0 |
| | **164** | 150 | 200 | 0 | 0 | 100 | 0 |
| **3** | **106** | 100 | 100 | 0 | 50 | 100 | 0 |
| | **108** | 100 | NS | 100 | 50 | 150 | 0 |
| | **110** | 150 | 50 | 150 | 100 | 150 | 50 |
| | **115** | 100 | 100 | 0 | 0 | 0 | 50 |
| | **117** | 250 | 0 | 0 | 50 | 200 | 200 |
| | **120** | 100 | 150 | 50 | 100 | 100 | 250 |
| | **122** | 350 | 100 | 300 | 150 | 250 | 200 |
| | **130** | 100 | 100 | 450 | 200 | 150 | 100 |
| | **131** | 150 | 200 | 300 | 350 | 200 | 50 |
| | **132** | 50 | 150 | 150 | 150 | 50 | 50 |
| | **144** | 200 | 200 | 150 | 100 | 150 | 50 |
| | **145** | 100 | 200 | 200 | 200 | 150 | 150 |
| | **147** | 100 | 200 | 200 | 150 | 250 | 100 |
| | **159** | 150 | 0 | 300 | 250 | 150 | 100 |
| | **162** | 200 | 150 | 150 | 100 | 300 | 150 |
| NS=No sample | | | | | | | |

*Conclusions.* Based on arithmetic mean FECs, the levamisole/eprinomectin injection had an efficacy of 90% or greater and this effect was maintained up to 43 days after treatment. DECTOMAX (doramectin) was insufficiently effective at all time points. Based on geometric mean FECs, the levamisole/eprinomectin injection was over 98% effective and this effect was maintained up to 43 days after treatment. DECTOMAX was over 86% effective during the same period. Efficacies were evaluated in animals carrying a mixed infection of *Ostertagia*, *Trichostrongylus and Cooperia* spp.

Example 12 - Efficacy of investigational eprinomectin / levamisole compared with doramectin-containing product

*Methods.* This study was performed in accordance with Example 11. The doses of IVP and CP were calculated according to individual body weights. Group 1 was treated with the IVP at the dose rate of 1mL/ 35 kg live weight; Group 2 was treated with the Control Product at the recommended dose rate of 1mL/50kg; and Group 3 was left untreated as the negative control.

*Results.* No injection site reactions were observed in Group 1 animals on either Day 17 or Day 28. Table 43 shows group arithmetic mean FEC at Day -6 and mean bodyweights at Day 0 and Day 56. Means in the same column that share a letter (A) are not significantly different ($p<0.05$).

Table 43. Group details

| Group | n | Mean (range) FEC Day-6 (epg) | Mean (range) bodyweight Day 0 (kg) | Mean (range) bodyweight Day 56 (kg) |
|---|---|---|---|---|
| 1 | 15 | $170^{A}$ (100-300) | 159 (129-189) | 184 (155-205) |
| 2 | 15 | $180^{A}$ (100-350) | 158 (136-209) | 183 (152.5-240) |
| 3 | 15 | $187^{A}$ (100-450) | 156 (127-197) | 178 (149-220) |

Table 44 shows group arithmetic and geometric mean FEC at Days 0-42. Means in the same column that do not share a letter (A/B) are significantly different ($p<0.05$). Based on geometric means, the FECs for Group 1 (treated with the IVP) were significantly less ($p<0.05$) than both the control group (Group 3) and the group treated with DECTOMAX (Group 2) on both of Days 16 and 28.

Table 44. Group FECs

| Group | Treatment | Arithmetic mean (range) FEC (epg) | | | |
|---|---|---|---|---|---|
| | | D 0 | D 16 | D 28 | D 42 |
| 1 | IVP | $503^{A}$ (0-1800) | $3^{B}$ (0-50) | $3^{B}$(0-50) | $60^{A}$ (0-200) |
| 2 | Dectomax | $393^{A}$ (0-1050) | $160^{A}$ (0-1050) | $103^{A}$ (0-700) | $86^{A}$ (0-200) |
| 3 | Control | $254^{A}$ (0-550) | $137^{AB}$ (0-600) | $125^{A}$ (0-250) | $83^{A}$ (0-150) |
| | | Geometric mean FEC (epg) | | | |
| | | D 0 | D 16 | D 28 | D 42 |
| 1 | IVP | $249.2^{A}$ | $0.3^{A}$ | $0.3^{A}$ | $17.6^{A}$ |
| 2 | Dectomax | $161.7^{A}$ | $28.1^{B}$ | $16.8^{B}$ | $35.8^{A}$ |
| 3 | Control | $137.8^{A}$ | $52.7^{B}$ | $68.0^{B}$ | $30.4^{A}$ |

The efficacy of the IVP and CP at each timepoint (based on FEC reduction compared to the untreated control group is shown in Table 45. The percentage of each nematode genus identified in 100 larvae from group coprocultures at each timepoint is shown in Table 46.

Table 45. Percentage FEC reduction efficacy compared to untreated control

| Group | Treatment | % Efficacy (arithmetic means) | | | % Efficacy (geometric means) | | |
|---|---|---|---|---|---|---|---|
| | | D 16 | D 28 | D 42 | D16 | D28 | D42 |
| 1 | IVP | 97.6 | 97.3 | 28 | 99.0 | 99.6 | 42.2 |
| 2 | Dectomax | -17.1 | 17.3 | -2.9 | 47.0 | 75.3 | -18.0 |

Table 46. Larval culture results (%)

| Day | Group | Treatment | Ostertagia | Trichostrongylus | Cooperia | Haemonchus |
|---|---|---|---|---|---|---|
| 0 | Pooled | NA | 12 | 8 | 80 | 0 |
| 16 | 1 | IVP | 97 | 0 | 3 | 0 |
| | 2 | Dectomax | 2 | 0 | 98 | 0 |
| | 3 | NA | 5 | 0 | 95 | 0 |
| 28 | 1 | IVP | 56 | 0 | 44 | 0 |
| | 2 | Dectomax | 11 | 0 | 89 | 0 |
| | 3 | NA | 23 | 9 | 68 | 0 |
| 42 | 1 | IVP | 2 | 0 | 98 | 0 |
| | 2 | Dectomax | 3 | 0 | 97 | 0 |
| | 3 | NA | 8 | 0 | 92 | 0 |

Table 47. Individual FEC results

| Group | ID | STRONGYLE FEC (epg) | | | | |
|---|---|---|---|---|---|---|
| | | D-6 | D0 | D16 | D28 | D42 |
| **1** | **77** | 150 | 50 | 50 | 50 | 0 |
| | **88** | 150 | 0 | 0 | 0 | 150 |
| | **91** | 200 | 1800 | 0 | 0 | 200 |
| | **94** | 300 | 850 | 0 | 0 | 50 |
| | **97** | 100 | 650 | 0 | 0 | 0 |
| | **102** | 200 | 150 | 0 | 0 | 100 |
| | **105** | 100 | 250 | 0 | 0 | 100 |
| | **112** | 150 | 200 | 0 | 0 | 0 |
| | **116** | 200 | 300 | 0 | 0 | 0 |
| | **119** | 150 | 400 | 0 | 0 | 50 |
| | **120** | 300 | 700 | 0 | 0 | 0 |
| | **123** | 200 | 1050 | 0 | 0 | 50 |
| | **124** | 100 | 100 | 0 | 0 | 50 |
| | **133** | 100 | 600 | 0 | 0 | 100 |
| | **136** | 150 | 450 | 0 | 0 | 50 |

(continued)

| Group | ID | STRONGYLE FEC (epg) | | | | |
|---|---|---|---|---|---|---|
| | | D-6 | D0 | D16 | D28 | D42 |
| 2 | 76 | 350 | 850 | 0 | 50 | 200 |
| | 82 | 100 | 650 | 100 | 0 | 200 |
| | 83 | 200 | 250 | 100 | 100 | 0 |
| | 85 | 150 | 350 | 100 | 100 | 100 |
| | 92 | 100 | 0 | 0 | 0 | 50 |
| | 98 | 150 | 200 | 100 | 50 | 0 |
| | 100 | 200 | 150 | 0 | 0 | 150 |
| | 103 | 200 | 250 | 200 | 150 | 100 |
| | 111 | 150 | 1050 | 1050 | 700 | 100 |
| | 121 | 100 | 850 | 400 | 200 | 50 |
| | 122 | 200 | 100 | 50 | 100 | 100 |
| | 127 | 150 | 0 | 0 | 0 | ns |
| | 129 | 150 | 250 | 100 | 0 | 100 |
| | 132 | 300 | 700 | 200 | 100 | 0 |
| | 137 | 200 | 250 | 0 | 0 | 50 |
| 3 | 78 | 150 | 250 | 100 | 150 | 0 |
| | 81 | 100 | 50 | 150 | 100 | 150 |
| | 84 | 150 | 200 | 100 | 250 | 100 |
| | 87 | 100 | 50 | 150 | 150 | 0 |
| | 90 | 150 | 400 | 100 | 100 | 100 |
| | 95 | 200 | 150 | 100 | 150 | 100 |
| | 99 | 450 | 150 | 100 | 100 | 100 |
| | 101 | 200 | 550 | 0 | 0 | 0 |
| | 104 | 100 | ns | 200 | 100 | 0 |
| | 106 | 150 | 0 | 100 | 100 | 50 |
| | 110 | 150 | 550 | 150 | 200 | 100 |
| | 117 | 400 | 350 | 200 | 200 | 150 |
| | 118 | 250 | 300 | 0 | ns | 100 |
| | 125 | 100 | 500 | 600 | 150 | 150 |
| | 128 | 150 | 50 | 0 | 0 | 150 |
| ns = no sample | | | | | | |

*Conclusions.* Based on arithmetic mean FECs, the levamisole/eprinomectin injection was over 97.6% effective and this effect was maintained up to 28 days after treatment. DECTOMAX was insufficiently effective at all time points. Based on geometric mean FECs, the levamisole / eprinomectin injection was over 99% effective and this effect was maintained up to 28 days after treatment. DECTOMAX was insufficiently effective at all time points. Efficacies were evaluated in animals carrying a mixed infection of *Ostertagia*, *Trichostrongylus* and *Cooperia spp.* There was strong evidence of resistance by *Cooperia spp.* to doramectin.

## Claims

1. A pharmaceutically or veterinarily acceptable formulation adapted to be injected into an animal, which formulation comprises;

   a. an effective amount of a levamisole;
   b. an effective amount of at least one macrocyclic lactone;
   c. a pharmaceutically acceptable system of solvents and surfactants wherein the system provides for acceptable storage stability for both the levamisole and the macrocyclic lactone and wherein the system provides for a viscosity which is suitable for injection;
   wherein the system comprises at least one surfactant, wherein the surfactant is CREMOPHOR EL,
   and a water miscible organic solvent which is dimethylacetamide (DMA).

2. The formulation of claim 1 wherein the levamisole is levamisole phosphate and wherein the levamisole is present in an amount sufficient to deliver a dose of at least 4 mg/kg animal bodyweight.

3. The formulation of claim 1, wherein the macrocyclic lactone is either ivermectin or eprinomectin and is present in an amount sufficient to deliver a dose of at least 150 $\mu$g/kg animal bodyweight.

4. The formulation of any one of claims 1 or 3 which further comprises an antioxidant, a preservative, and water for injection wherein the water for injection is provided in a quantity sufficient to bring the formulation to a suitable injection volume.

5. The formulation of any one of claims 1 to 4 wherein the macrocyclic lactone concentration is from about 0.1% to about 2% (w/v); the levamisole concentration is from about 15% to about 30% (w/v); and the surfactant concentration is from about 1% to about 10% (w/v).

6. The formulation of claim 5 wherein the macrocyclic lactone is eprinomectin and its concentration is about 0.5% to about 0.9% (w/v); the levamisole is levamisole phosphate and its concentration is about 18% to about 25% (w/v); the surfactant is CREMOPHOR EL, or a combination of CREMOPHOR EL and POLYSORBATE 80, and its concentration is about 3% to about 7% (w/v); the antioxidant is BHT; the solvent is DMA and its concentration is about 1% to about 10% (w/v); and the preservative is methyl paraben.

7. The formulation of any one of claims 1-6 wherein the macrocyclic lactone is eprinomectin and wherein the eprinomectin has a specification such that its B1a composition is greater than or equal to 85.0% and its B1a + B1b composition is greater than or equal to 90.0%; and wherein the density of the formulation is between 0.95 and 1.20 and pH is between 3.0 - 5.0.

8. A pharmaceutically or veterinarily acceptable formulation adapted to be injected into an animal, which formulation comprises;

   a. an effective amount of a levamisole;
   b. an effective amount of at least one macrocyclic lactone;
   c. a pharmaceutically acceptable system of solvents and surfactants wherein the system provides for acceptable storage stability for both the levamisole and the macrocyclic lactone and wherein the system provides for a viscosity which is suitable for injection;
   wherein the system comprises at least one surfactant, wherein the surfactant is CREMOPHOR EL,
   and a water miscible organic solvent which is dimethylacetamide (DMA), wherein the macrocyclic lactone is eprinomectin and its concentration is about 0.5% to about 0.9% (w/v); the levamisole is levamisole phosphate and its concentration is about 18% to about 25% (w/v); the surfactant is POLYSORBATE 80, CREMOPHOR EL, or combinations thereof, and its concentration is about 3% to about 7% (w/v); the antioxidant is BHT, the solvent is DMA and its concentration is about 1% to about 10% (w/v); and the preservative is methyl paraben.

9. A formulation according to any one of claims 1 to 8 for use in preventing or treating internal parasite infestations in animals.

10. A formulation for use according to claim 9 wherein the formulation is injected.

11. A formulation for use according to claim 9 or 10 wherein the macrocyclic lactone concentration is from 0.5% to 0.9% (w/v) and the levamisole concentration is from 15% to 25% (w/v).

12. A formulation for use according to claim 9 or 10 wherein the parasites are helminths.

13. A formulation for use according to claim 12 wherein the helminths are nematodes, cestodes, trematodes.

14. A method for producing the formulation according to claim 1 or 2 comprising the steps of:

a. adding water for injection to a vessel,
b. dissolving the Levamisole in the water,
c. in a separate vessel dissolving Eprinomectin and BHT in DMA,
d. adding the surfactant to the DMA/Eprinomectin blend,
e. transferring the Eprinomectin/DMA blend to the bulk aqueous phase with mixing, and
f. optionally, bringing the batch to volume with added WFI and mix.

## Patentansprüche

1. Pharmazeutisch oder veterinärmedizinisch verträgliche Formulierung, die angepasst ist, um in ein Tier injiziert zu werden, wobei die Formulierung umfasst:

a. eine wirksame Menge an einem Levamisol;
b. eine wirksame Menge an mindestens einem makrocyclischen Lacto n ;
c. ein pharmazeutisch verträgliches System an Lösungsmitteln und Tensiden, wobei das System eine verträgliche Lagerungsstabilität sowohl für das Levamisol und das makrocyclische Lacton bereitstellt und wobei das System eine Viscosität bereitstellt, die zur Injektion geeignet ist;
und wobei das System mindestens ein Tensid umfasst, wobei das Tensid CREMOPHOR EL ist, und ein wassermischbares organisches Lösungsmittel, das Dimethylacetamid (DMA) ist.

2. Formulierung nach Anspruch 1, wobei das Levamisol Levamisol-Phosphat ist und wobei das Levamisol in einer Menge vorliegt, die ausreicht, um eine Dosis von mindestens 4 mg/kg Tierkörpergewicht zu verabreichen.

3. Formulierung nach Anspruch 1, wobei das makrocyclische Lacton entweder Ivermectin oder Eprinomectin ist und in einer Menge vorliegt, die ausreicht, um eine Dosis von mindestens 150 $\mu$g/kg Tierkörpergewicht zu verabreichen.

4. Formulierung nach einem der Ansprüche 1 oder 3, die des Weiteren ein Antioxidans, ein Konservierungsmittel und Wasser zur Injektion umfasst, wobei das Wasser zur Injektion in einer Menge vorhanden ist, die ausreicht, um die Formulierung auf ein geeignetes Injektionsvolumen zu bringen.

5. Formulierung nach einem der Ansprüche 1 bis 4, wobei die makrocyclische Lactonkonzentration von etwa 0,1% bis etwa 2% (w/v) ist, die Levamisolkonzentration von etwa 15% bis etwa 30% (w/v) ist; und die Tensidkonzentration von etwa 1% bis etwa 10% (w/v) ist.

6. Formulierung nach Anspruch 5, wobei das makrocyclische Lacton Eprinomectin ist und seine Konzentration etwa 0,5% bis etwa 0,9% (w/v) ist; das Levamisol Levamisol-Phosphat ist und seine Konzentration etwa 18% bis 25% (w/v) ist; das Tensid CREMOPHOR EL, oder eine Kombination von CREMOPHOR EL und POLYSORBAT 80 ist und seine Konzentration etwa 3% bis etwa 7% (w/v) ist; das Antioxidans BHT ist; das Lösungsmittel DMA ist und seine Konzentration etwa 1% bis etwa 10% (w/v) ist; und das Konservierungsmittel Methylparaben ist.

7. Formulierung nach einem der Ansprüche 1 bis 6, wobei das makrocyclische Lacton Eprinomectin ist und wobei wobei Eprinomectin die Spezifikation hat, dass sein Gehalt an B1 a größer oder gleich 85,0 % und sein Gehalt an B1a + B1b größer oder gleich 90,0% ist; und wobei die Dichte der Formulierung zwischen 0,95 und 1,20 ist und der pH-Wert zwischen 3,0 bis 5,0 ist.

8. Pharmazeutisch oder veterinärmedizinisch verträgliche Formulierung, die angepasst ist, um in ein Tier injiziert zu werden, wobei die Formulierung umfasst:

a. eine wirksame Menge an einem Levamisol;
b. eine wirksame Menge an mindestens einem makrocyclischen Lacto n ;
c. ein pharmazeutisch verträgliches System an Lösungsmitteln und Tensiden, wobei das System eine verträgliche Lagerungsstabilität sowohl für das Levamisol und das makrocyclische Lacton bereitstellt und wobei das System eine Viscosität bereitstellt, die zur Injektion geeignet ist;
und wobei das System mindestens ein Tensid umfasst, wobei das Tensid CREMOPHOR EL ist, und ein wassermischbares organisches Lösungsmittel, das Dimethylacetamid (DMA) ist, wobei das makrocyclische Lacton Eprinomectin ist und seine Konzentration etwa 0,5% bis etwa 0,9% (w/v) ist; das Levamisol Levamisol-Phosphat ist und seine Konzentration etwa 18% bis etwa 25% (w/v) ist; das Tensid POLYSORBAT 80, CREMOPHOR EL oder eine Kombination davon ist und seine Konzentration etwa 3% bis etwa 7% ist; das Antioxidans BHT ist, das Lösungsmittel DMA ist und seine Konzentration etwa 1% bis etwa 10% (w/v) ist; und das Konservierungsmittel Methylparaben ist.

**9.** Formulierung nach einem der Ansprüche 1 bis 8, zur Verwendung bei der Vorbeugung oder Behandlung von innerlichem Parasitenbefall bei Tieren.

**10.** Formulierung zur Verwendung nach Anspruch 9, wobei die Formulierung injiziert wird.

**11.** Formulierung zur Verwendung nach Anspruch 9 oder 10, wobei die makrocyclische Lactonkonzentration von 0,5% bis 0,9% (w/v) ist und die Levamisolkonzentration von 15% bis 25% (w/v) ist.

**12.** Formulierung zur Verwendung nach Anspruch 9 oder 10, wobei die Parasiten Helminthen sind.

**13.** Formulierung zur Verwendung nach Anspruch 12, wobei die Helminthen Nematoden, Cestoden, Trematoden sind.

**14.** Verfahren zur Herstellung der Formulierung nach Anspruch 1 oder 2, umfassend die Schritte:

a. Hinzufügen von Wasser zur Injektion in ein Gefäß,
b. Lösen des Levamisols in dem Wasser,
c. Lösen von Eprinomectin und BHT in DMA in einem separaten Gefäß,
d. Hinzufügen des Tensids zu der DMA/Eprinomectin-Mischung,
e. Transferieren der DMA/Eprinomectin-Mischung in die wässrige Bulk-Phase durch Mischen, und
f. gegebenenfalls die Charge durch das Wasser zur Injektion (WFI) bis zum Injektionsvolumen auffüllen und Mischen.

**Revendications**

**1.** Formulation de qualité pharmaceutique ou vétérinaire conçue pour être injectée à un animal, ladite formulation comprenant :

a. une quantité efficace de lévamisole ;
b. une quantité efficace d'au moins une lactone macrocyclique ;
c. un système de solvants et de tensioactifs de qualité pharmaceutique, dans laquelle le système présente une stabilité acceptable au stockage à la fois pour le lévamisole et pour la lactone macrocyclique, et dans laquelle le système a une viscosité qui est appropriée pour une injection ;
dans laquelle le système comprend au moins un tensioactif, dans laquelle le tensioactif est le CREMOPHOR EL, et un solvant organique miscible avec l'eau qui est le diméthylacétamide (DMA).

**2.** Formulation selon la revendication 1, dans laquelle le lévamisole est le phosphate de lévamisole et dans laquelle le lévamisole est présent en une quantité suffisante pour administrer une dose d'au moins 4 mg/kg de poids corporel de l'animal.

**3.** Formulation selon la revendication 1, dans laquelle la lactone macrocyclique est soit l'ivermectine soit l'éprinomectine et est présente en une quantité suffisante pour administrer une dose d'au moins 150 μg/kg de poids corporel de l'animal.

**4.** Formulation selon l'une quelconque des revendications 1 ou 3, qui comprend en outre un antioxydant, un conservateur, et de l'eau pour l'injection, dans laquelle l'eau pour l'injection est prévue en une quantité suffisante pour amener la formulation à un volume d'injection approprié.

**5.** Formulation selon l'une quelconque des revendications 1 à 4, dans laquelle la concentration en lactone macrocyclique est située dans la plage allant d'environ 0,1 % à environ 2 % (p/v) ; la concentration en lévamisole est située dans la plage allant d'environ 15 % à environ 30 % (p/v) ; et la concentration en tensioactif est située dans la plage allant d'environ 1 % à environ 10 % (p/v).

**6.** Formulation selon la revendication 5, dans laquelle la lactone macrocyclique est l'éprinomectine et sa concentration est située dans la plage allant d'environ 0,5 % à environ 0,9 % (p/v) ; le lévamisole est le phosphate de lévamisole et sa concentration est située dans la plage allant d'environ 18 % à environ 25 % (p/v) ; le tensioactif est le CREMOPHOR EL, ou une combinaison de CREMOPHOR EL et de POLYSORBATE 80, et sa concentration est située dans la plage allant d'environ 3 % à environ 7 % (p/v) ; l'antioxydant est le BHT ; le solvant est le DMA et sa concentration est située dans la plage allant d'environ 1 % à environ 10 % (p/v) ; et le conservateur est le p-hydroxybenzoate de méthyle.

**7.** Formulation selon l'une quelconque des revendications 1 à 6, dans laquelle la lactone macrocyclique est l'éprinomectine et dans laquelle l'éprinomectine a une spécification telle que sa composition en constituant B1a est supérieure ou égale à 85,0 % et que sa composition en constituants B1a + B1b est supérieure ou égale à 90,0 % ; et dans laquelle la densité de la formulation est comprise entre 0,95 et 1,20 et le pH est compris entre 3,0 et 5,0.

**8.** Formulation de qualité pharmaceutique ou vétérinaire conçue pour être injectée à un animal, ladite formulation comprenant :

a. une quantité efficace de lévamisole ;
b. une quantité efficace d'au moins une lactone macrocyclique ;
c. un système de solvants et de tensioactifs de qualité pharmaceutique, dans laquelle le système présente une stabilité acceptable au stockage à la fois pour le lévamisole et pour la lactone macrocyclique, et dans laquelle le système a une viscosité qui est appropriée pour une injection ;
dans laquelle le système comprend au moins un tensioactif, dans laquelle le tensioactif est le CREMOPHOR EL, et un solvant organique miscible avec l'eau qui est le diméthylacétamide (DMA), dans laquelle la lactone macrocyclique est l'éprinomectine et sa concentration est située dans la plage allant d'environ 0,5 % à environ 0,9 % (p/v) ; le lévamisole est le phosphate de lévamisole et sa concentration est située dans la plage allant d'environ 18 % à environ 25 % (p/v) ; le tensioactif est le POLYSORBATE 80, le CREMOPHOR EL, ou des combinaisons de ceux-ci, et sa concentration est située dans la plage allant d'environ 3 % à environ 7 % (p/v) ; l'antioxydant est le BHT, le solvant est le DMA et sa concentration est située dans la plage allant d'environ 1 % à environ 10 % (p/v) ; et le conservateur est le p-hydroxybenzoate de méthyle.

**9.** Formulation selon l'une quelconque des revendications 1 à 8, destinée à être utilisée dans la prévention ou le traitement d'infestations d'animaux par des endoparasites.

**10.** Formulation destinée à être utilisée selon la revendication 9, dans laquelle la formulation est injectée.

**11.** Formulation destinée à être utilisée selon la revendication 9 ou la revendication 10, dans laquelle la concentration en lactone macrocyclique est située dans la plage allant de 0,5 % à 0,9 % (p/v) et la concentration en lévamisole est située dans la plage allant de 15 % à 25 % (p/v).

**12.** Formulation destinée à être utilisée selon la revendication 9 ou la revendication 10, dans laquelle les parasites sont des helminthes.

**13.** Formulation destinée à être utilisée selon la revendication 12, dans laquelle les helminthes sont des nématodes, des cestodes, des trématodes.

**14.** Procédé de production de la formulation selon la revendication 1 ou la revendication 2, comprenant les étapes suivante :

a. ajouter de l'eau pour l'injection dans un récipient,
b. dissoudre le lévamisole dans l'eau,

c. dans un récipient séparé, dissoudre l'éprinomectine et le BHT dans le DMA,
d. ajouter le tensioactif au mélange DMA/éprinomectine,
e. transférer le mélange éprinomectine/DMA à la phase aqueuse mère tout en mélangeant, et
f. éventuellement, amener le lot au volume souhaité avec un ajout d'eau pour l'injection et sous mélange.

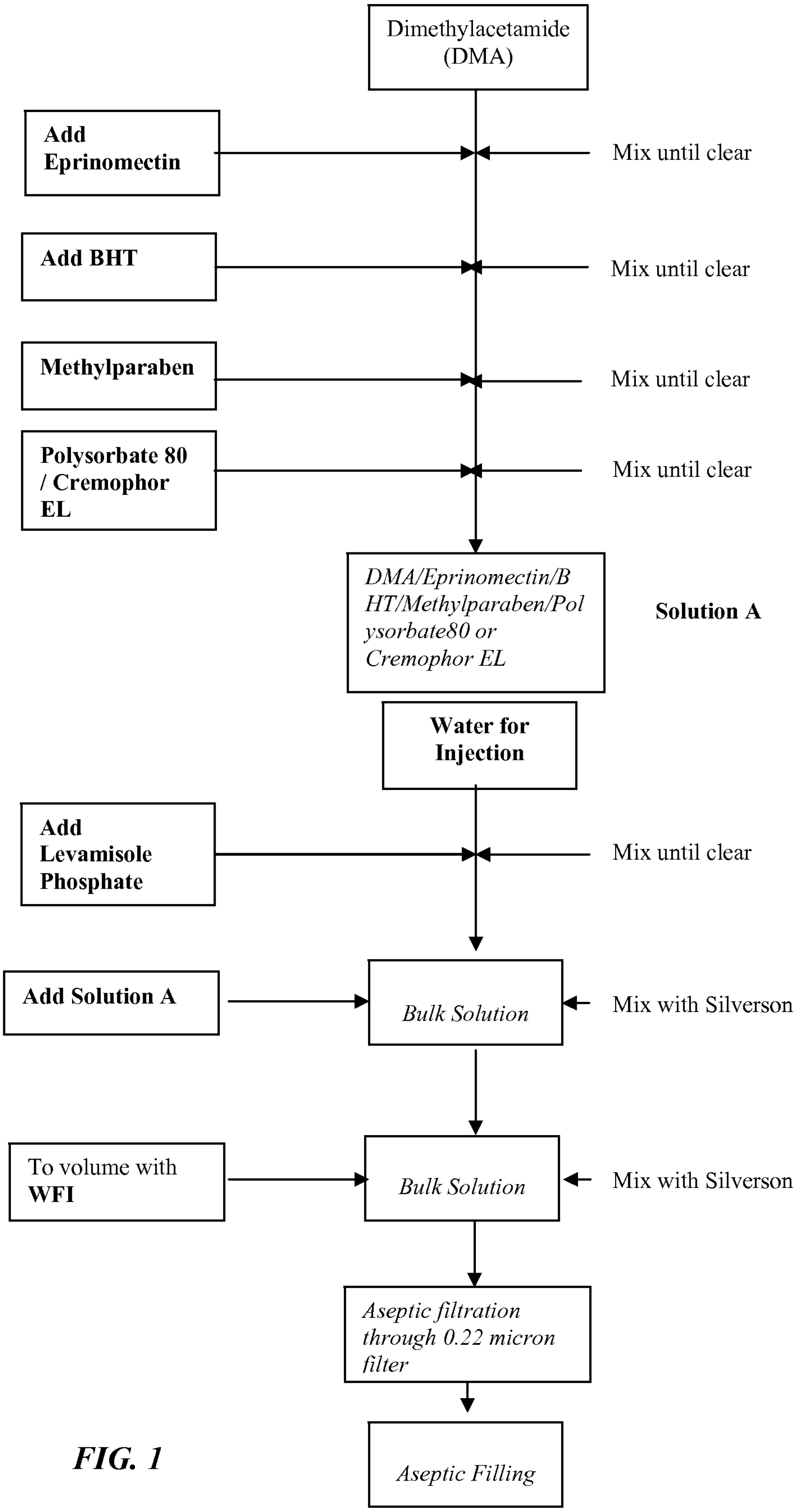
Dimethylacetamide (DMA)
Add Eprinomectin
Mix until clear
Add BHT
Mix until clear
Methylparaben
Mix until clear
Polysorbate 80 / Cremophor EL
Mix until clear
DMA/Eprinomectin/BHT/Methylparaben/Polysorbate80 or Cremophor EL
Solution A
Water for Injection
Add Levamisole Phosphate
Mix until clear
Add Solution A
Bulk Solution
Mix with Silverson
To volume with WFI
Bulk Solution
Mix with Silverson
Aseptic filtration through 0.22 micron filter
Aseptic Filling

*FIG. 1*

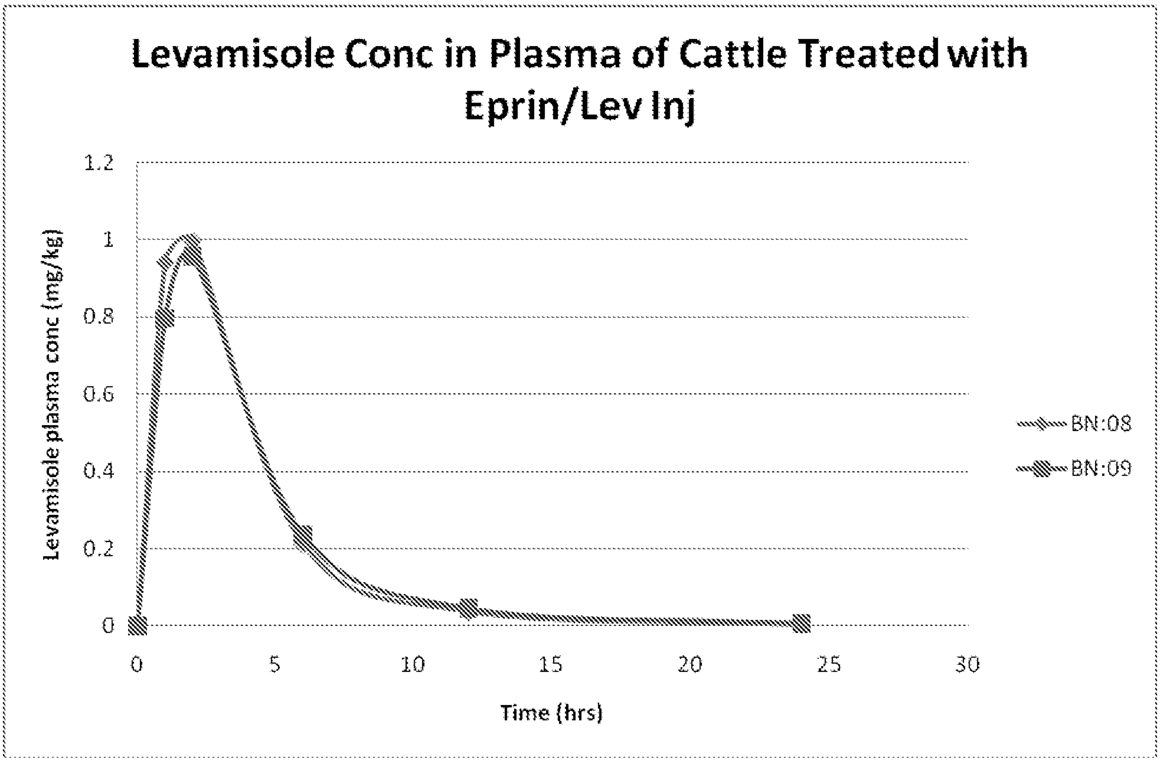
Levamisole Conc in Plasma of Cattle Treated with Eprin/Lev Inj
Levamisole plasma conc (mg/kg)
1.2
1
0.8
0.6
0.4
0.2
0
0
5
10
15
20
25
30
Time (hrs)
BN:08
BN:09

*FIG. 2*

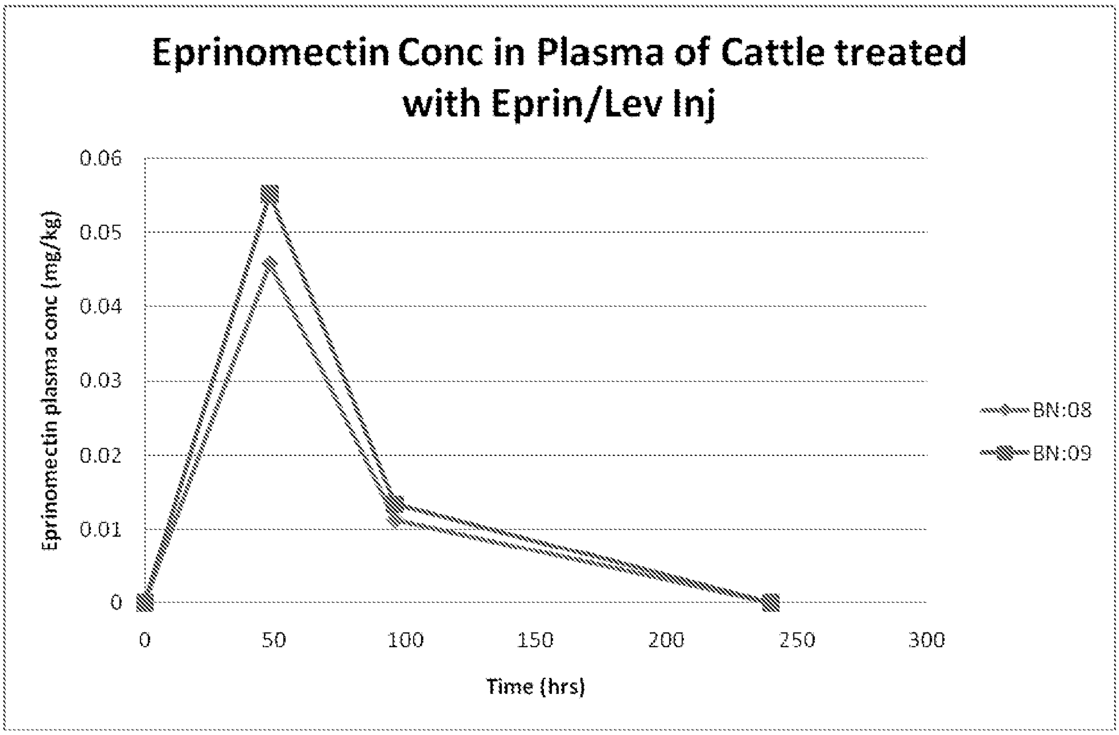
Eprinomectin Conc in Plasma of Cattle treated with Eprin/Lev Inj
Eprinomectin plasma conc (mg/kg)
0.06
0.05
0.04
0.03
0.02
0.01
0
0
50
100
150
200
250
300
Time (hrs)
BN:08
BN:09

*FIG. 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61333882 A
- US 4310519 A, Albers-Schonberg
- US 4199569 A, Chabala
- US 3950360 A, Aoki
- US 5077308 A
- US 4859657 A
- US 4963582 A
- US 4855317 A
- US 4871719 A
- US 4874749 A
- US 4427663 A
- US 5055596 A
- US 4973711 A
- US 4978677 A
- US 4920148 A
- US 6174540 B
- US 6733767 B
- US 5602107 A
- US 20090075918 A1, Neto
- WO 0074489 A
- WO 2007068073 A
- WO 2004069242 A
- WO 00061068 A
- WO 04009080 A
- WO 2010021555 A
- AU 200310102, Nufarm and Novartis
- US 4381397 A
- US 7704961 B
- US 7521429 B
- US 20060105970 A1
- US 7678740 B
- US 7632820 B
- US 7678773 B
- US 7605134 B
- US 6933260 B


### Non-patent literature cited in the description


- Ivermectin and Abamectin. Springer-Verlag, 1989
- The Merck Index. Merck & Co., Inc, 1996
- **SARI et al.** *Journal of liquid chromatography and related technologies,* 2006, vol. 29 (15), 2277-2290